# EUROPÄISCHE PATENTANMELDUNG

(11) **EP 3 791 831 A1**
(43) Veröffentlichungstag der Anmeldung: **17.03.2021**
(21) Anmeldenummer: 19196499.8
(22) Anmeldetag: 10.09.2019
(51) Int. Cl.: A61F 2/44, A61F 2/46, B33Y 80/00

(54) **IMPLANTAT ZUR BEHANDLUNG EINER BANDSCHEIBE**

(71) Anmelder: AIT Austrian Institute of Technology GmbH, 1210 Wien (AT)
(72) Erfinder: Die Erfindernennung liegt noch nicht vor
(74) Vertreter: Wirnsberger & Lerchbaum Patentanwälte OG

(57) **Zusammenfassung**

Die Erfindung betrifft ein Implantat (1) zur Behandlung einer Bandscheibe (6), indem zumindest ein Teil der Bandscheibe (6) durch zumindest einen Teil des Implantates (1) ersetzt wird und/oder zumindest ein Teil des Implantates (1) in die Bandscheibe (6) eingeführt wird, wobei das Implantat (1) einen Erstreckungskörper aufweist, welcher von einem Positionierungszustand in einen Erstreckungszustand, in welchem der Erstreckungskörper in zumindest einer Richtung, insbesondere quer zu einer Einführrichtung des Implantates (1), eine größere Länge aufweist als im Positionierungszustand, überführbar ist, um den Erstreckungskörper im Positionierungszustand zumindest teilweise in einem Bandscheibenzwischenwirbelraum der Bandscheibe (6) zu positionieren und anschließend in den Erstreckungszustand zu überführen. Um eine hohe Belastbarkeit und hohe Biokompatibilität zu erreichen, ist erfindungsgemäß vorgesehen, dass der Erstreckungskörper mit einer bioresorbierbaren Mg-Zn-Ca-Legierung gebildet ist.

Weiter betrifft die Erfindung ein Verfahren zur Herstellung eines derartigen Implantates.

## Beschreibung

Die Erfindung betrifft ein Implantat zur Behandlung einer Bandscheibe, indem zumindest ein Teil der Bandscheibe durch zumindest einen Teil des Implantates ersetzt wird und/oder zumindest ein Teil des Implantates in die Bandscheibe eingeführt wird, wobei das Implantat einen Erstreckungskörper aufweist, welcher von einem Positionierungszustand in einen Erstreckungszustand, in welchem der Erstreckungskörper in zumindest einer Richtung, insbesondere quer zu einer Einführrichtung des Implantates, eine größere Länge aufweist als im Positionierungszustand, überführbar ist, um den Erstreckungskörper im Positionierungszustand zumindest teilweise in einem Bandscheibenzwischenwirbelraum der Bandscheibe zu positionieren und anschließend in den Erstreckungszustand zu überführen.

Des Weiteren betrifft die Erfindung ein Verfahren zur Herstellung eines derartigen Implantates.

Bandscheiben, auch Zwischenwirbelscheiben genannt, bilden als Teile einer Wirbelsäule eine tragende Struktur der Wirbelsäule. Eine Bandscheibe ist dabei in der Regel zwischen zwei Wirbelkörpern zweier benachbarter Wirbel der Wirbelsäule angeordnet, wobei der Bandscheibe insbesondere eine Funktionalität einer Kräfteverteilung und Dämpfung von auf die Wirbelsäule bzw. Wirbel wirkenden Kräfte zukommt. Ein üblicherweise von der Bandscheibe zwischen benachbarten Wirbeln eingenommener Raum wird als Bandscheibenzwischenwirbelraum der Bandscheibe bezeichnet. Eine Bandscheibe umfasst dabei typischerweise einen äußeren Faserring, als Anulus-Fibrosus (Anulus fibrosus) bezeichnet, und einen Gallertkern, als Nucleus-Pulposus (Nucleus pulposus) bezeichnet, wobei der Nucleus-Pulposus üblicherweise zentral gelegen in der Bandscheibe angeordnet ist und vom Anulus-Fibrosus insbesondere ringförmig umfasst ist. An den beiden an die benachbarten Wirbelkörper der Bandscheibe angrenzenden Seiten der Bandscheibe ist die Bandscheibe - normalerweise mit Ausnahme einer Außenzone des Anulus-Fibrosus - mit hyalinknorpeligen Deckplatten der Wirbelkörper abgeschlossen bzw. bedeckt. Der Anulus-Fibrosus ist im Regelfall mit konzentrischen Schichten von kollagenen Bindegewebsfasern (Typ-1-Kollagenfasern) gebildet, welche in Richtung Zentrum der Bandscheibe in faserknorpeliges Gewebe (Typ-2-Kollagenfasern) übergehen, welches faserknorpelige Gewebe für gewöhnlich außerdem mit den hyalinknorpeligen Deckplatten in Verbindung steht. Der Nucleus-Pulposus weist einen mehrheitlichen Anteil von Wasser auf, häufig zwischen 70 % und 90 %, welches mit gallertigem Gewebe des Nucleus-Pulposus reversibel gebunden ist. Auf diese Weise erklärt sich eine druckelastische bzw. stoßdämpfende Funktionalität des Nucleus-Pulposus bzw. der Bandscheibe bei kurzzeitigen Belastungen, da dem Nucleus-Pulposus Eigenschaften einer Nicht-Kompressibilität bei gleichzeitiger Verformbarkeit zugeschrieben werden können, vergleichbar mit einem Wasserkissen. Bei üblichen Belastungen der Wirbelsäule können dabei in der Bandscheibe häufig Drücke, auch als intradiskale Drücke bezeichnet, von bis zu 23 bar auftreten. Bei langzeitigen Belastungen der Bandscheibe gibt der Nucleus-Pulposus reversibel Flüssigkeit bzw. Wasser ab, was mit einer Höhenabnahme der Bandscheibe verbunden ist. Bei anschließender reduzierter Belastung der Bandscheibe wird Flüssigkeit bzw. Wasser vom Nucleus-Pulposus wieder aufgenommen und gebunden. Eine derartige reversible Flüssigkeitsabgabe und Aufnahme stellt üblicherweise außerdem eine Basis für eine Nährstoffversorgung bzw. einen Stoffwechsel der Bandscheibe dar.

Im Falle eines pathologischen Befundes der Bandscheibe, beispielsweise bei einem Bandscheibenvorfall, bei welchem Teile der Bandscheibe in Folge eines Risses im Anulus-Fibrosus in einen Wirbelkanal vortreten, ist es bekannt, Implantate zur Behandlung der Bandscheibe anzuwenden, welche in die Bandscheibe eingeführt bzw. eingesetzt werden und/oder einen Teil der Bandscheibe ersetzen.

Ein bekanntes Verfahren zur Behandlung einer solchen Bandscheibe umfasst dabei eine zumindest teilweise Entnahme des Nucleus-Pulposus bzw. von Nucleus-Pulposus-Material aus der Bandscheibe und ein Einfügen eines mit einem expandierbaren Körper, beispielsweise einem Ballon, gebildeten Implantates in die Bandscheibe bzw. deren Bandscheibenzwischenwirbelraum, um durch Expandieren des expandierbaren Körpers, beispielsweise durch Einfüllen eines Füllmediums unter Druck in einen Hohlraum des expandierbaren Körpers, einen üblichen intradiskalen Druck in der Bandscheibe bzw. deren Bandscheibenzwischenwirbelraum besser zu verteilen und insbesondere einen Wirbelabstand einzustellen bzw. auf einen weniger pathologischen Abstand zu vergrößern.

So offenbart das Dokument WO 2007/025164 A2 ein Implantat zum Ersatz eines Nucleus-Pulposus, wobei ein expandierbarer Ballon in einem komprimierten Zustand in eine Bandscheibe eingebracht und anschließend durch Einfüllen eines flüssigen Füllmediums in den Ballon expandiert wird, um eine Funktionalität des Nucleus-Pulposus wiederherzustellen.

An einen Aufbau und eine Ausgestaltung eines Bandscheibenimplantates werden dabei in der Regel hohe Anforderungen gestellt, zumal das Bandscheibenimplantat typischerweise wechselnden hohen Belastungen standhalten muss, andererseits gleichzeitig eine ausgeprägte Biokompatibilität aufweisen soll. Dies gilt im besonderen Maße für ein Bandscheibenimplantat, welches mit einem expandierbaren Körper gebildet ist und entsprechend zusätzlich eine zuverlässige Expansionsfähigkeit gewährleisten muss.

Besonderes Augenmerk ist dabei im Besonderen auf eine Biokompatibilität eines in ein biologisches System einer Bandscheibe eingebrachten Implantates bzw. dessen Materials zu legen, zumal das Implantat in der Regel einen Fremdkörper im biologischen System darstellt. Eine elegante Möglichkeit eine Biokompatibilität von Bandscheibenimplantaten zu optimieren, wird durch eine Verwendung von bioresorbierbaren Materialien als Implantatmaterialien erreicht, welche sich mit der Zeit im biologischen System einer Bandscheibe auflösen und von diesem resorbiert bzw. absorbiert werden. Dies ermöglicht es, gegebenenfalls auf weitere operative Eingriffe, beispielsweise zur Entfernung eines Implantates oder von Implantatteilen, zu verzichten und bietet darüber hinaus das Potenzial, ein Degradationsverhalten bzw. Degradierungsverhalten des Implantates auf einen Heilungs- bzw. Regenerierungsprozess der Bandscheibe abzustimmen.

Bekannt geworden sind Versuche, ein Bandscheibenimplantat bzw. dessen expandierbaren Körper aus Poly(lactid-co-glycolid), auch PLGA genannt, zu bilden, um auf diese Weise einen in eine Bandscheibe einfügbaren expandierbaren Körper zu schaffen, welcher den hohen Druckbelastungen in einer Bandscheibe zumindest zeitbegrenzt standhält, gleichzeitig bioresorbierbar ist und zudem eine Expansionsfähigkeit aufweist, welche eine strukturelle Belastungsfähigkeit des expandierbaren Körpers nicht zu sehr unterminiert. Ein Nachteil von biodegradierbaren Polymerstrukturen ist eine im Vergleich zu Metallen geringere Festigkeit und Belastbarkeit. Darüber hinaus ist ein Degradationsprozess von aus Polymerstrukturen gebildeten Implantatteilen häufig mit einer Volumenzunahme der Implantatteile verbunden, da der Dergradierungsprozess unter ausgeprägter Hydrolyse stattfindet.

Hier setzt die Erfindung an. Aufgabe der Erfindung ist es, ein Implantat der eingangs genannten Art anzugeben, welches eine hohe Belastbarkeit und hohe Biokompatibilität aufweist.

Weiter ist es ein Ziel der Erfindung, ein Verfahren zur Herstellung eines solchen Implantates anzugeben.

Die Aufgabe wird erfindungsgemäß dadurch gelöst, dass der Erstreckungskörper mit einer bioresorbierbaren Mg-Zn-Ca-Legierung gebildet ist.

Grundlage der Erfindung ist die Idee, in Bezug auf den Erstreckungskörper einen Kompromiss zwischen einander üblicherweise teilweise entgegenstehenden Einzelwirkungen einer hohen Belastbarkeit, insbesondere hohen Festigkeit, ausgeprägten Duktilität sowie außerdem vorhandener Bioresorbierbarkeit zu finden, sodass eine hohe Robustheit bzw. Belastbarkeit, insbesondere bei gleichzeitig ermöglichter Änderung einer Gestalt des Erstreckungskörpers erhalten ist, um ein entsprechendes Implantat mit Erstreckungskörper für die besonderen Bedingungen innerhalb einer Bandscheibe umzusetzen. Es hat sich gezeigt, dass dies mit einer bioresorbierbaren Mg-Zn-Ca-Legierung aufgrund derer mechanischen Eigenschaften ermöglicht ist. Durch eine Verwendung einer Metalllegierung, im Speziellen einer Mg-Zn-Ca-Legierung, welche sich für den betreffenden Zweck als besonders geeignet herausgestellt hat, können ausgeprägte Festigkeiten auch von dünnen Bauteilstrukturen erreicht werden. Weiter enthält bzw. besteht diese Legierung im Wesentlichen aus Elementen, welche in menschlichen oder tierischen Systemen grundsätzlich bereits vorhanden sind, wodurch Reiz- und Reaktionserscheinungen in hohem Maße vermeidbar sind. Darüber hinaus hat sich gezeigt, dass eine bioresorbierbare Mg-Zn-Ca-Legierung innerhalb eines biologischen Milieus einer Bandscheibe praktisch vollständig abbaubar ist. Es hat sich schließlich herausgestellt, dass eine solche Mg-Zn-Ca-Legierung im Besonderen geeignet ist, expandierbare Körper, insbesondere in Form von Hüllen bzw. Ummantelungen, herzustellen, sodass beispielsweise ballonartige oder schlauchförmige Hüllkörper mit, insbesondere aus der Mg-Zn-Ca-Legierung gebildet werden können, um den expandierbaren Körper im Positionierungszustand zumindest teilweise an einer Einsatzposition in einem Bandscheibenzwischenwirbelraum der Bandscheibe - also in einem üblicherweise von der Bandscheibe zwischen benachbarten Wirbeln eingenommenen Raum - bzw. in der Bandscheibe zu positionieren und anschließend in den Erstreckungszustand zu überführen bzw. zu expandieren, um insbesondere einem Druck innerhalb der Bandscheibe, üblicherweise einem intradiskalen Druck bis etwa 23 bar, standzuhalten. Indem der Erstreckungskörper zumindest teilweise, bevorzugt gänzlich, aus einer bioresorbierbaren Mg-Zn-Ca-Legierung gebildet ist, ist somit eine hohe Belastbarkeit und hoher Biokompatibilität des Implantates erreichbar.

Mit Vorteil ist vorgesehen, dass der Erstreckungskörper in dessen Positionierungszustand zumindest teilweise, insbesondere gänzlich, durch eine Öffnung in einem Anulus-Fibrosus der Bandscheibe in einen Hohlraum der Bandscheibe einfügbar ist, um nach anschließendem Überführen des Erstreckungskörpers in dessen Erstreckungszustand mit dem expandierbaren Körper einen Nucleus-Pulposus der Bandscheibe zumindest teilweise, insbesondere gänzlich, zu ersetzen und/oder die Öffnung im Anulus-Fibrosus zu verschließen. Auf diese Weise ist eine besonders schonende Behandlung der Bandscheibe erreichbar. Dabei ist in einer üblichen Herangehensweise vorgesehen, dass die Öffnung im Anulus-Fibrosus in Form zumindest eines Bohrloches, häufig mehrerer Bohrlöcher, in den Anulus-Fibrosus eingebracht ist bzw. wird, durch welche insbesondere ein Teil oder der gesamte Nucleus-Pulposus der Bandscheibe entfernt bzw. entnommen wird. Zweckmäßig kann dann der Erstreckungskörper auf vorgenannte Weise in die Bandscheibe bzw. einen Hohlraum der Bandscheibe zumindest teilweise, insbesondere gänzlich, eingefügt werden. Ein solcher Hohlraum der Bandscheibe, in welchem der Erstreckungskörper zumindest teilweise, insbesondere gänzlich, einfügbar ist, kann sowohl durch die vorgenannte Entnahme des Nucleus-Pulposus als auch durch das in den Anulus-Fibrosus eingebrachte Bohrloch gebildet sein. Der Erstreckungskörper kann somit ausgebildet sein, zumindest teilweise, insbesondere gänzlich, in einen Bandscheibenbereich des Nucleus-Pulposus eingefügt zu werden, um den Nucleus-Pulposus zumindest teilweise, insbesondere gänzlich, zu ersetzen, oder in ein Bohrloch im Anulus-Fibrosus eingefügt zu werden, um eine durch das Bohrloch gebildete Öffnung im Anulus-Fibrosus zu verschließen oder beides. Ein übliches in den Anulus-Fibrosus eingebrachtes Bohrloch weist dabei einen, insbesondere maximalen, Durchmesser bzw. eine, insbesondere maximale, Querschnittsbreite von mehreren mm, meist zwischen 2 mm und 20 mm, insbesondere zwischen 2 mm und 10 mm, bevorzugt zwischen 2 mm und 8 mm, insbesondere bevorzugt zwischen 2 mm und 5 mm, auf. Zweckmäßig ist es somit, wenn der Erstreckungskörper im Positionierungszustand eine Form und Ausdehnung aufweist, um zumindest teilweise, insbesondere gänzlich, in bzw. durch ein solcherart ausgebildetes Bohrloch eingefügt bzw. durchgeführt zu werden. Praktikabel ist es, wenn der Erstreckungskörper in dessen Erstreckungszustand in zumindest einer Richtung, insbesondere einer Richtung quer bzw. winklig, insbesondere orthogonal, zu einer Einführrichtung des Implantates in den Hohlraum der Bandscheibe eine größere Länge aufweist als im Positionierungszustand. Einführrichtung bezeichnet dabei üblicherweise jene Richtung, in welcher das Implantat bzw. der Erstreckungskörper in die Bandscheibe bzw. einen Bandscheibenzwischenwirbelraum bzw. einen Hohlraum der Bandscheibe eingefügt wird. Vorteilhaft für eine robuste Positionierung des Implantates ist es, wenn die Länge dabei zumindest gleich groß oder größer ist als ein Durchmesser des Bohrloches bzw. die Öffnung im Anulus-Fibrosus. Vorgenanntes ist mit einem Erstreckungskörper, welcher mit, insbesondere aus, einer Mg-Zn-Ca-Legierung, im Besonderen gemäß nachstehend dargelegter Legierungszusammensetzung, gebildet ist, erreichbar. In der Regel wird versucht, einen Durchmesser bzw. eine Querschnittsbreite des Bohrloches möglichst klein zu halten und gleichzeitig das Implantat bzw. den Erstreckungskörper derart auszubilden, dass dieser in dessen Erstreckungszustand den Hohlraum der Bandscheibe möglichst ausfüllt bzw. insbesondere darüber hinaus eine gewünschte Druckverteilung innerhalb der Bandscheibe bewirkt.

Bewährt hat es sich, dass der Erstreckungskörper mit einem expandierbaren Körper ausgebildet ist, welcher im Erstreckungszustand ein größeres Volumen aufweist als im Positionierungszustand. Praktikabel kann damit der Erstreckungskörper bzw. expandierbare Körper von dessen Positionierungszustand in dessen Erstreckungszustand, in welchem der expandierbare Körper ein größeres Volumen aufweist als im Positionierungszustand, überführt bzw. expandiert werden, um den expandierbaren Körper im Positionierungszustand zumindest teilweise in einem Bandscheibenzwischenwirbelraum bzw. Hohlraum der Bandscheibe zu positionieren und anschließend in den Erstreckungszustand zu expandieren. Insbesondere kann der Erstreckungskörper als expandierbarer Körper ausgebildet sein. Wie vorstehend dargelegt, hat es sich gezeigt, dass mit einer bioresorbierbaren Mg-Zn-Ca-Legierung Hohlkörper, insbesondere ballonartige oder schlauchförmige Hüllkörper, mit veränderbarer bzw. expandierbarer Gestalt bzw. Form herstellbar sind, welche den hohen Belastungen in einer Bandscheibe nachhaltig standhalten können. Indem der expandierbare Körper mit einer bioresorbierbaren Mg-Zn-Ca-Legierung gebildet ist, ist eine hohe Robustheit bzw. Belastbarkeit bei insbesondere hoher Einsatzpraktikabilität des Implantates erreichbar, wobei eine hohe Bioverträglichkeit gegeben ist. Der expandierbare Körper kann teilweise oder bevorzugt gänzlich mit der bioresorbierbaren Mg-Zn-Ca-Legierung ausgebildet sein.

Expandierbarer Körper bezeichnet dabei insbesondere einen Körper, dessen Volumen gezielt durch eine auf ein Material des expandierbaren Körpers wirkende bzw. aufgebrachte Kraft veränderbar ist, und ist somit im Besonderen in Abgrenzung zu einer üblichen jedem Körper immanenten Befähigung zu einer thermischen Expansion basierend auf einem thermischen Ausdehnungskoeffizienten zu sehen, sodass eine solche damit begrifflich nicht umfasst bzw. bezeichnet ist. Insbesondere bezeichnet Volumen des expandierbaren Körpers dabei das Volumen, welches der expandierbare Körper aufgrund dessen Gestalt bzw. Form, insbesondere in der Bandscheibe bzw. im Bandscheibenzwischenwirbelraum, einnimmt bzw. beansprucht.

Günstig ist es, wenn der expandierbare Körper zumindest einen mit einem fließfähigen, insbesondere flüssigen, gelartigen und/oder gasförmigen, Füllmedium befüllbaren Füllraum aufweist, um den expandierbaren Körper durch Befüllen des Füllraumes mit Füllmedium vom Positionierungszustand in den Erstreckungszustand zu überführen. Auf diese Weise ist ein besonders einfach zu handhabendes Implantat umsetzbar. Insbesondere kann dadurch der expandierbare Körper im Positionierungszustand sehr kompakt ausgebildet und im Erstreckungszustand gezielt, insbesondere abgestimmt auf einen mit dem expandierbaren Körper einzunehmendem Volumenbereich, auf eine gewünschte Größe expandiert werden. Bewährt hat es sich, wenn der expandierbare Körper hierzu eine ballonartige, kissenartige oder schlauchartige Hülle bzw. Ummantelung aufweist oder bildet, mit welcher der Füllraum gebildet und insbesondere füllmediumsdicht, im Speziellen flüssigkeitsdicht und/oder gasdicht, ummantelt ist. Vorteilhaft ist es, wenn der expandierbare Körper mehrere vorgenannte Füllräume aufweist, welche insbesondere separat voneinander mit Füllmedium befüllbar sind. Dies ermöglicht eine robuste bzw. belastbare Ausgestaltung des Füllkörpers und erleichtert insbesondere eine Positionierung des expandierbaren Körpers an dessen Einsatzposition bzw. im Bandscheibenzwischenwirbelraum. Je nach Anwendungsbedingungen kann es günstig sein, wenn die mehreren Füllräume räumlich derart verbunden sind, dass Füllmedium von einem Füllraum zu einem anderen Füllraum gelangen bzw. strömen kann, beispielsweise indem zwei oder einige oder sämtliche Füllräume mit Verbindungsleitungen verbunden sind. Es kann aber auch günstig sein, wenn der expandierbare Körper mehrere räumlich voneinander getrennte Füllräume aufweist, sodass ein in einen Füllraum eingebrachtes Füllmedium nicht in einen anderen Füllraum gelangen bzw. strömen kann.

Durch Zuführen von Füllmedium, üblicherweise in Form einer bevorzugt biokompatiblen Flüssigkeit und/oder einem bevorzugt biokompatiblen Gel, in den Hohlraum der Bandscheibe und/oder im Besonderen einem oder mehreren Füllräumen des expandierbaren Körpers kann auf belastbare Weise eine Funktionalität der Bandscheibe zumindest teilweise wieder hergestellt werden und insbesondere ein intradiskaler Druck in der Bandscheibe gleichmäßig verteilt werden.

Ein einfaches Befüllen des Hohlraumes der Bandscheibe bzw. des Füllraumes bzw. der Füllräume des expandierbaren Körpers ist praktikabel umsetzbar, wenn der Erstreckungskörper, insbesondere der expandierbare Körper und/oder nachstehend erläuterter Basiskörper, eine oder mehrere Füllöffnungen aufweist, über welche der Hohlraum der Bandscheibe, insbesondere der Füllraum bzw. die Füllräume des expandierbaren Körpers, mit Füllmedium befüllbar sind. Weist der expandierbare Körper mehrere Füllräume auf, ist ein effizientes Befüllen der Füllräume erreichbar, wenn unterschiedlichen Füllräumen unterschiedliche Füllöffnungen zugeordnet sind, sodass die Füllräume separat voneinander mit Füllmedium befüllbar sind. Auf diese Weise können, insbesondere unterschiedliche, Füllmedien in unterschiedliche Füllräume, im Besonderen zeitparallel, eingebracht werden. Das Füllmedium ist üblicherweise als fließfähiges, insbesondere gasförmiges und/oder flüssiges und/oder gelartiges, Medium ausgebildet.

Vorteilhaft ist es, wenn der Erstreckungskörper einen Basiskörper und zumindest einen relativ zum Basiskörper beweglichen, insbesondere schwenkbaren bzw. kippbaren, Blockierkörper aufweist, um den Erstreckungskörper durch Bewegen, insbesondere Schwenken, des Blockierkörpers relativ zum Basiskörper vom Positionierungszustand in den Erstreckungszustand zu überführen. Damit ist eine flexible und praktikable Handhabung des Implantates ermöglicht. Dies ist zweckmäßig umsetzbar, indem durch Bewegen, insbesondere Schwenken, des Blockierkörpers relativ zum Basiskörper eine Länge des Erstreckungskörpers, üblicherweise in zumindest einer Richtung quer bzw. winklig, insbesondere orthogonal, zur einer Einführrichtung des Implantates vergrößerbar ist, um den Erstreckungskörper vom Positionierungszustand in den Erstreckungszustand zu überführen. Praktikabel kann damit der Erstreckungskörper von dessen Positionierungszustand in dessen Erstreckungszustand, in welchem der Erstreckungskörper eine größere Länge aufweist als im Positionierungszustand, überführt werden, um den Erstreckungskörper im Positionierungszustand zumindest teilweise in einem Bandscheibenzwischenwirbelraum bzw. Hohlraum der Bandscheibe zu positionieren und anschließend in den Erstreckungszustand zu überführen. Es versteht sich, dass der Blockierkörper je nach Einsatzbedingungen mit unterschiedlicher Größe bzw. Form bzw. Länge ausgebildet sein kann, insbesondere um mit dem Blockierkörper einen Nucleus-Pulposus der Bandscheibe zumindest teilweise zu ersetzen und/oder die Öffnung im Anulus-Fibrosus zu verschließen und/oder ein Festsetzen des Erstreckungskörpers im Bandscheibenzwischenwirbelraum bzw. dem Hohlraum der Bandschiebe zu unterstützen, insbesondere umzusetzen. Üblicherweise ist dabei vorgesehen, dass der Blockierkörper von einer Positionierungseinstellung in zumindest eine Erstreckungseinstellung, vorteilhaft in mehrere Erstreckungseinstellungen, relativ zum Basiskörper bewegbar, insbesondere schwenkbar bzw. kippbar, ist, sodass in der Erstreckungseinstellung des Blockierkörpers eine Länge des Erstreckungskörpers in zumindest einer Richtung, insbesondere quer bzw. winklig, bevorzugt orthogonal, zu einer Einführrichtung des Implantates, größer ist als in der Positionierungseinstellung des Blockierkörpers. Die Positionierungseinstellung des Blockierkörpers korrespondiert dabei mit dem Positionierungszustand des Erstreckungskörpers und die Erstreckungseinstellung des Blockierkörpers korrespondiert mit dem Erstreckungszustand des Erstreckungsköpers. Es versteht sich, dass es vorteilhaft ist, wenn unterschiedliche Erstreckungseinstellungen des Blockierkörpers unterschiedlichen Längen des Erstreckungskörpers entsprechen. Je nach Anwendungsbedingungen kann es günstig sein, wenn ein Bewegen, insbesondere Schwenken bzw. Kippen, des Blockierkörpers von der Positionierungseinstellung in eine oder mehrere Erstreckungseinstellungen, insbesondere reversibel, also umkehrbar, oder nicht reversibel, also nicht umkehrbar, durchführbar ist. Dadurch kann die Länge des Erstreckungskörpers abgestimmt auf jeweilige Einsatzbedingungen gewählt werden.

Für eine hohe Robustheit ist es günstig, wenn der Blockierkörper in der bzw. den Erstreckungseinstellung(en) arretierbar ist. Praktikabel kann hierfür eine Arretierungseinrichtung vorhanden sein, um den Blockierkörper, insbesondere formschlüssig und/oder kraftschlüssig, in einer Erstreckungseinstellung zu arretieren. Eine hohe Anwendungsflexibilität ist erreichbar, wenn der Erstreckungskörper sowohl mit einem expandierbaren Körper als auch mit einem vorgenannten relativ zum Basiskörper bewegbaren Blockierkörper gebildet ist. Insbesondere kann hierzu der Blockierkörper mit einem expandierbaren Körper bzw. als expandierbarer Körper ausgebildet sein. Es können auch mehrere vorgenannte Blockierkörper am Basiskörper vorhanden bzw. in vorgenannter Weise mit diesem verbunden sein.

Günstig ist es, wenn der Blockierkörper mit einem Gelenk mit dem Basiskörper relativ zum Basiskörper bewegbar bzw. schwenkbar verbunden ist. Dies ermöglicht eine einfache Ausbildung des Erstreckungskörpers und ein verlässliches Bewegen bzw. Verschwenken des Blockierkörpers relativ zum Basiskörper. Zweckmäßig kann das Gelenk beispielsweise mit einem bzw. als Drehgelenk und/oder Schubgelenk ausgebildet sein. Eine hohe Einsatzpraktikabilität ist erreichbar, wenn der Blockierkörper und der Basiskörper mit dem Gelenk derart verbunden sind, dass der Blockierkörper um einen Kippwinkel von der Positionierungseinstellung in die Erstreckungseinstellung schwenkbar ist, wobei vorzugsweise ein maximaler Kippwinkel zwischen Blockierkörper und Basiskörper mit bzw. durch einen Anschlag am Implantat bzw. am Erstreckungskörper festgelegt ist. Eine hohe Flexibilität ist erreichbar, wenn der Blockierkörper, insbesondere mit der Arretierungseinrichtung, bei mehreren Kippwinkeln arretierbar ist. Für eine hohe Verlässlichkeit und Robustheit hat es sich bewährt, dass das Gelenk als Scharnier ausgebildet ist, welches bevorzugt mit einer in zumindest einer Drehachsenaufnahme drehbar bzw. schwenkbar gelagerten Drehachse gebildet ist. Die Drehachse kann dabei Teil des Basiskörpers und die Drehachsenaufnahme Teil des Basiskörpers sein, oder umgekehrt.

Für eine einfache Handhabung hat es sich bewährt, wenn der Blockierkörper durch Anstoßen mit einem, insbesondere von einem Benutzer bedienten bzw. geführten, Bedienungselement, beispielsweise einem Applikator, von der Positionierungseinstellung in die Erstreckungseinstellung überführbar ist. Das Bedienelement bzw. der Applikator ist dabei meist als Stab ausgebildet, welcher gegen das Blockierelement gedrückt bzw. gestoßen wird, um den Blockierkörper von der Positionierungseinstellung in die Erstreckungseinstellung zu überführen bzw. relativ zum Basiskörper zu schwenken. Für eine praktikable Bedienung ist es zweckmäßig, wenn das Implantat eine Bedienelementdurchführung aufweist, durch welche das Bedienelement zum Blockierkörper führbar ist, um diesen mit dem Bedienelement von der Positionierungseinstellung in die Erstreckungseinstellung zu überführen. Die Bedienelementdurchführung kann beispielsweise Teil einer bzw. mit einer, insbesondere nachstehend erklärten, Mediumzufuhrleitung und/oder Füllöffnung und/oder Mediumzufuhrdurchführung des Basiskörpers gebildet sein, über welche gegebenenfalls Füllmedium dem Hohlraum der Bandscheibe bzw. dem Füllraum zuführbar ist. Günstig ist es, wenn der Blockierkörper ein Stoßsegment aufweist und derart mit dem Basiskörper verbunden ist, dass der Blockierkörper durch Drücken gegen das Stoßsegment, beispielsweise mit einem insbesondere von einem Benutzer geführten, Bedienelement bzw. Applikator, von dessen Positionierungseinstellung in dessen Erstreckungseinstellung überführbar ist. Das Drücken ist effizient und/oder kontinuierliche durchführbar, wenn das Stoßsegment als Erhebung einer Außenoberfläche des Blockierkörpers ausgebildet ist, wobei die Außenoberfläche des Stoßsegmentes vorzugsweise mit einem bzw. als Abschnitt einer Rotationsfläche ausgebildet ist.

Um abgestimmt auf jeweilige Einsatzbedingungen eine hohe Bioverträglichkeit sicherzustellen, kann der Basiskörper und/oder der Blockierkörper teilweise oder bevorzugt gänzlich mit der bioresorbierbaren Mg-Zn-Ca-Legierung ausgebildet sein.

Bewährt hat es sich, dass das Implantat zumindest eine mit dem Erstreckungskörper, insbesondere expandierbaren Körper bzw. Basiskörper und/oder Blockierkörper, verbundene Mediumzufuhrleitung aufweist, über welche Füllmedium dem Hohlraum der Bandscheibe bzw. gegebenenfalls dem zumindest einen Füllraum, gegebenenfalls mehreren Füllräumen, insbesondere über die eine oder mehreren Füllöffnungen, zuführbar ist. Damit kann auf einfache Weise Füllmedium dem Hohlraum der Bandscheibe bzw. dem expandierbaren Körper bzw. dem einen oder mehreren Füllräumen des expandierbaren Körpers zugeführt werden. Dies ist praktikabel umsetzbar, indem die Mediumzufuhrleitung an die Füllöffnung oder mehreren Füllöffnungen des Erstreckungskörpers, insbesondere expandierbaren Körpers und/oder Basiskörpers, anschließt. Üblicherweise ist die Füllöffnung durch eine Mediumzufuhrdurchführung des Erstreckungskörpers, insbesondere expandierbaren Körpers bzw. Basiskörpers, gebildet, über welche Füllmedium dem Hohlraum der Bandscheibe bzw. Füllraum zuführbar ist. Für eine effiziente Befüllung ist es günstig, wenn das Implantat mehrere derartige mit dem Erstreckungskörper bzw. expandierbaren Körper verbundene Mediumzufuhrleitungen aufweist. Günstig ist es, wenn unterschiedliche Mediumzufuhrleitungen an unterschiedliche Füllräume bzw. unterschiedliche Füllöffnungen des expandierbaren Körpers anschließen, also unterschiedlichen Füllräumen zugeordnet sind. Dadurch ist ein flexibles und anwendungsfallabhängiges Befüllen ermöglicht, insbesondere wenn unterschiedliche Füllmedien verwendet werden. Zur Befüllung des Hohlraumes der Bandscheibe bzw. des Implantates bzw. des Erstreckungskörpers, insbesondere expandierbaren Körpers, in der Regel während einer Bandscheibenoperation bzw. Bandscheibenbehandlung, kann dann ein Mediumzufuhrschlauch an die Mediumzufuhrleitung des Implantates angesteckt bzw. mit dieser verbunden werden, um ein Medium durch den Mediumzufuhrschlauch über die Mediumzufuhrleitung dem Erstreckungskörper, insbesondere expandierbaren Körper, zuzuführen, und nach Abschluss der Befüllung wieder von der Mediumzufuhrleitung des Implantates entfernt werden. Ein robuster Aufbau ist erreichbar, wenn die Mediumzufuhrleitung als rohrförmiges, bevorzugt starres, Element ausgebildet ist.

Grundsätzlich können der Erstreckungskörper, insbesondere der expandierbare Körper bzw. der Basiskörper und/oder Blockierkörper, und die Mediumzufuhrleitung formschlüssig und/oder kraftschlüssig oder stoffschlüssig miteinander verbunden bzw. verbindbar sein. Eine belastbare Verbindung zwischen Mediumzufuhrleitung und Erstreckungskörper ist erreichbar, wenn die Mediumzufuhrleitung mittels kraftschlüssiger Verbindung, bevorzugt einer Klemmverbindung, mit dem Erstreckungskörper bzw. expandierbaren Körper, insbesondere lösbar, verbunden bzw. verbindbar ist. Dies ist etwa umsetzbar, indem die Mediumzufuhrleitung mittels eines Klemmelementes, beispielsweise eines Klemmringes, mit dem Erstreckungskörper verbunden bzw. verbindbar ist. Hierzu kann vorgesehen sein, dass ein Randbereich einer vorgenannten Füllöffnung des Erstreckungskörpers bzw. expandierbaren Körpers über einen Abschnitt der Mediumzufuhrleitung stülpbar und kraftschlüssig, beispielsweise mit dem Klemmelement, insbesondere Klemmring, mit der Mediumzufuhrleitung verbindbar ist. Mit einem üblichen Klemmring ist dabei auf einfache Weise eine Quetschverbindung durch kraftschlüssiges Aneinanderpressen eines Abschnittes des Erstreckungskörpers bzw. expandierbaren Körpers, beispielsweise eines Randbereiches einer Füllöffnung des Erstreckungskörpers bzw. expandierbaren Körpers, mit einem Abschnitt der Mediumzufuhrleitung erreichbar. Wenngleich meist etwas aufwendiger, ist es alternativ oder kumulativ praktikabel umsetzbar, dass die Mediumzufuhrleitung mittels formschlüssiger Verbindung mit dem Erstreckungskörper, insbesondere expandierbaren Körper bzw. Basiskörper, insbesondere lösbar, verbunden bzw. verbindbar ist.

Vorteilhaft ist es, wenn die Mediumzufuhrleitung und der Erstreckungskörper bzw. expandierbare Körper mit einem Schnellverschluss miteinander, insbesondere lösbar, verbunden sind. Ein Schnellverschluss ermöglicht es, eine Verbindungseinrichtung schnell, insbesondere ohne Werkzeug zu lösen bzw. zu schließen. Auf einfache Weise ist ein Schnellverschluss für eine formschlüssige Verbindung, also eine Verbindung durch räumlichen Einschluss, dadurch umgesetzt, dass der räumliche Einschluss durch ein bewegbares Verschlusselement aufgehoben bzw. wiederhergestellt werden kann. Günstig ist es, wenn ein Schnappverschluss vorhanden ist, um eine formschlüssige oder kraftschlüssige Verbindung zwischen der Mediumzufuhrleitung und dem Erstreckungskörper bzw. expandierbaren Körper herzustellen. Dabei ist üblicherweise vorgesehen, dass die Verbindung herstellbar ist, indem ein Schnappelement elastisch verformt wird und anschließend einen Formschluss oder Kraftschluss bewirkt. Durch abermaliges elastisches Verformen des Schnappelementes ist der Formschluss bzw. Kraftschluss wieder aufhebbar. Eine solche Verbindung ist auf einfache Weise schnell verbindbar und gegebenenfalls wieder lösbar. Denkbar ist aber auch, dass eine Verbindung in Form einer Schraubverbindung oder einer ähnlich zweckmäßigen Verbindungsform umgesetzt ist.

Eine Verbesserung einer Heilung der Bandscheibe ist erreichbar, wenn im und/oder am Implantat, insbesondere im und/oder am Erstreckungskörper bzw. in der und/oder an der Mediumzufuhrleitung, vorzugsweise in einem in einer Einsatzposition des Implantates der Bandscheibe zugewandten Randbereich dieses, heilungsfördernde Substanzen, beispielsweise Wachstumsfaktoren, Stammzellen und/oder autologe Zellen, angeordnet bzw. anordenbar sind. Zweckmäßig ist es, wenn das Implantat, insbesondere der Erstreckungskörper und/oder die Mediumzufuhrleitung ein oder mehrere Reservoire aufweisen, in welchen die heilungsfördernden Substanden angeordnet bzw. anordenbar sind. Die heilungsfördernden Substanzen können bei einem Herstellungsprozess des Implantates und/oder während der Operation, insbesondere in-situ, in das Implantat, bevorzugt das bzw. die Reservoir(e), eingebracht bzw. an diesem angeordnet werden. Insbesondere wenn der Implantatteil, in bzw. an welchem die heilungsfördernden Substanzen bzw. die Reservoire angeordnet sind, mit einem bioresorbierbaren Material gebildet ist, können die Substanzen, insbesondere sukzessive, während einer Degradation des Materials abgegeben werden. Vor allem wenn die heilungsfördernden Substanzen in vorgenannter Weise in bzw. an der Mediumzufuhrleitung angeordnet sind, kann eine schnelle Heilung des Anulus-Fibrosus erreicht werden, um dadurch einen schnellen Verschluss des Bohrloches bzw. der Öffnung im Anulus-Fibrosus zu erreichen.

Es hat sich bewährt, dass das Implantat zumindest ein steuerbares Durchflussventil aufweist, um eine Durchflussmenge, insbesondere einen Volumenstrom, eines über die Füllöffnung des Erstreckungskörpers, insbesondere expandierbaren Körpers bzw. Basiskörpers, dem Hohlraum der Bandscheibe bzw. Füllraum des expandierbaren Körpers zugeführten Füllmediums zu steuern. Praktikabel ist es, wenn das Durchflussventil an der Mediumzufuhrleitung und/oder dem Erstreckungskörper, insbesondere dem expandierbaren Körper bzw. Basiskörper, angeordnet ist, um eine Durchflussmenge, insbesondere einen Volumenstrom, eines über die Mediumzufuhrleitung und/oder eine Füllöffnung des Erstreckungskörpers bzw. expandierbaren Körpers dem Füllraum zugeführten Füllmediums zu steuern. Eine zweckmäßige Umsetzung ist erreichbar, wenn das Durchflussventil ein Durchflusssteuerelement und ein relativ zum Erstreckungskörper, insbesondere expandierbaren Körper bzw. Basiskörper, bzw. der Mediumzufuhrleitung bewegliches Durchflussventilelement aufweist, wobei das Durchflussventilelement einen Durchflussquerschnitt der Füllöffnung und/oder der Mediumzufuhrleitung in Abhängigkeit einer Einstellung des Durchflusssteuerelementes ändert, um die Durchflussmenge, insbesondere den Volumenstrom, zu ändern. Ein robuster Aufbau ist dabei erreichbar, wenn das Durchflussventilelement mit einer mechanischen Verbindung, vorzugsweise starr, mit dem Durchflusssteuerelement verbunden ist, sodass durch ein Bewegen des Durchflusssteuerelementes relativ zur Mediumzufuhrleitung bzw. dem Erstreckungskörper, insbesondere expandierbaren Körper, das Durchflussventilelement relativ zur Mediumzufuhrleitung bzw. dem Erstreckungskörper, insbesondere expandierbaren Körper, bewegt wird, um die Durchflussmenge, insbesondere den Volumenstrom, zu ändern. Beispielsweise kann das Durchflussventilelement mit einer oder mehrerer Führungsstangen mit dem Durchflusssteuerelement verbunden sein, wobei die Führungsstangen beweglich in einem oder mehreren Führungslager(n) der Mediumzufuhrleitung gelagert sind, sodass durch Bewegen des Durchflusssteuerelementes das Durchflussventilelement bewegt wird, um die Durchflussmenge, insbesondere den Volumenstrom, zu ändern bzw. zu steuern. Praktikabel ist es, wenn das Durchflussventilelement als Verschlusspfropfen ausgebildet ist, um durch teilweises, insbesondere auch gänzliches, Verschließen der Mediumzufuhrleitung bzw. der Füllöffnung die Durchflussmenge bzw. den Volumenstrom zu ändern. Zweckmäßig ist es, wenn das Durchflusssteuerelement, derart ausgebildet und angeordnet ist, dass dieses von einem Bediener, beispielsweise einem Chirurgen, insbesondere leicht zugänglich betätigbar ist, um ein Befüllen des Hohlraumes der Bandscheibe bzw. des expandierbaren Füllkörpers mit Füllmedium zu steuern. Bewährt hat es sich hierzu, wenn das Durchflusssteuerelement in Mediumzufuhrrichtung der Mediumzufuhrleitung vorgelagert am Implantat angeordnet ist. Zweckmäßigerweise kann das Durchflusssteuerelement mit einem oder als Griff ausgebildet sein, um eine Betätigung durch einen Benutzer zu erleichtern. Der Griff kann dabei beispielsweise als, vorzugsweise bewegliches, Applikatorelement, etwa als Kolben, oder mit einem anderen dem Fachmann bekannten Betätigungselement ausgebildet sein.

Günstig ist es, wenn das Implantat zumindest eine Rückschlageinrichtung aufweist, welche ein Zuführen von Füllmedium über eine Füllöffnung des Erstreckungskörpers, insbesondere expandierbaren Körpers bzw. Basiskörpers, in den Hohlraum der Bandscheibe bzw. einem oder mehreren Füllräumen des expandierbaren Körpers erlaubt, aber einen Austritt von Füllmedium aus dem Hohlraum, insbesondere Füllraum bzw. Füllräumen, über die Füllöffnung begrenzt oder verhindert. Praktikabel ist es, wenn die Rückschlageinrichtung an der Mediumzufuhrleitung und/oder dem Erstreckungskörper, insbesondere dem expandierbaren Körper bzw. Basiskörper, angeordnet ist, sodass ein Zuführen von Füllmedium über die Mediumzufuhrleitung und/oder eine Füllöffnung des Erstreckungskörpers, insbesondere expandierbaren Körpers bzw. Basiskörpers, in den Hohlraum, insbesondere den Füllraum oder die Füllräume, erlaubt, aber ein Austritt von Füllmedium aus dem Hohlraum bzw. dem Füllraum oder den Füllräumen, insbesondere über die Mediumzufuhrleitung und/oder die Füllöffnung, begrenzt oder verhindert ist. Eine zweckmäßige Umsetzung ist erreichbar, indem die Rückschlageinrichtung ein relativ zum Erstreckungskörper, insbesondere expandierbaren Körper bzw. Basiskörper, bzw. der Mediumzufuhrleitung bewegliches Rückschlagverschlusselement aufweist, welches die Füllöffnung und/oder die Mediumzufuhrleitung verschließt, wenn eine von einem im Hohlraum bzw. Füllraum befindlichen Füllmedium verursachte auf ein mit dem Rückschlagverschlusselement in Verbindung stehendes Kraftaufnahmeelement wirkende Druckkraft größer ist als eine von einem in Zuführrichtung zugeführten bzw. fließenden Füllmedium verursachte auf das Kraftaufnahmeelement wirkende Druckkraft, und insbesondere in einem umgekehrten Fall öffnet. Hierbei kann das Kraftaufnahmeelement das Rückschlagverschlusselement oder ein Teil davon sein. Auf diese Weise ist verhinderbar, dass ein in den Hohlraum der Bandscheibe bzw. den expandierbaren Körper bzw. dessen Füllraum eingefülltes Füllmedium ungewollt aus dem Hohlraum bzw. Füllraum über die Mediumzufuhrleitung bzw. die Füllöffnung wieder zurückfließt, beispielsweise wenn eine Befüllung des Hohlraumes bzw. Füllraumes beendet wird oder ist. Zweckmäßig ist es hierfür, wenn das Rückschlagverschlusselement einen oder mehrere Führungsstäbe aufweist, welche beweglich in Führungsaufnahmen der Mediumzufuhrleitung gelagert sind, sodass das Rückschlagverschlusselement relativ zur Mediumzufuhrleitung beweglich ist, wobei das Rückschlagverschlusselement die Mediumzufuhrleitung verschließt, wenn eine von einem im Hohlraum bzw. Füllraum befindlichen Füllmedium verursachte auf das Rückschlagverschlusselement wirkende Druckkraft größer ist als eine von einem in Zuführrichtung zugeführten bzw. fließenden Füllmedium verursachte auf das Rückschlagverschlusselement wirkende Druckkraft, und insbesondere in einem umgekehrten Fall öffnet. Druckkraft bezeichnet dabei insbesondere eine durch ein Füllmedium bzw. dessen Druck verursachte, im Besonderen auf das Kraftaufnahmeelement bzw. das Rückschlagverschlusselement wirkende, Kraft. Zweckmäßig ist es, wenn ein vorgenanntes Durchflussventil gleichzeitig als Rückschlageinrichtung ausgebildet ist, beispielsweise indem das Durchflussventilelement als Rückschlagverschlusselement ausgebildet ist.

Eine besonders hohe Biokompatibilität ist erreichbar, wenn die Mediumzufuhrleitung und/oder gegebenenfalls das Durchflussventil und/oder die Rückschlageinrichtung, bevorzugt das gesamte Implantat, mit, insbesondere aus, einem bioresorbierbaren Material, insbesondere einer bioresorbierbaren Mg-Zn-Ca-Legierung, gebildet ist. Damit ist erreichbar, dass ein besonders hoher Anteil des Implantates, bevorzugt das gesamte Implantat, mit bzw. aus einem Material gebildet ist, welches von einem menschlichen oder tierischen biologischen System abbaubar ist. Nachdem die Mediumzufuhrleitung und/oder das Durchflussventil und/oder die Rückschlageinrichtung in der Regel an einer Einsatzposition des Implantates kleineren Belastungen ausgesetzt sind als der Erstreckungskörper, ist es meist ausreichend, wenn diese mit bzw. aus einem üblichen bioresorbierbaren Material, wie beispielsweise mit bzw. aus Polylactid, auch als PLA bezeichnet, oder einem Derivat davon gebildet sind. Insbesondere Poly-L-Lactid, auch als PLLA bezeichnet, oder Poly(lactid-co-glycolid), auch als PLGA bezeichnet, haben sich aufgrund derer ausgeprägten mechanischen Festigkeiten als besonders geeignet herausgestellt. Für eine hohe Robustheit bzw. hohe Belastbarkeit ist es jedoch bevorzugt, wenn die Mediumzufuhrleitung und/oder gegebenenfalls das Durchflussventil und/oder die Rückschlageinrichtung mit, insbesondere aus, einer bioresorbierbaren Mg-Zn-Ca-Legierung gebildet ist, dies gilt im besonderen Maß für die Mediumzufuhrleitung.

Sowohl eine hohe Robustheit, Belastbarkeit und hohe Biokompatibilität des Implantates, insbesondere des Erstreckungskörpers, im Speziellen des expandierbaren Körpers bzw. Basiskörpers und/oder Blockierkörpers, vor allem in dessen Erstreckungszustand, ist erreichbar, wenn die bioresorbierbare Mg-Zn-Ca-Legierung (in Gew.-%) mehr als 0,0 %, insbesondere mehr als 0,1 %, bis 1,5 % Zink, mehr als 0,0 %, insbesondere mehr als 0,1 %, bis 1,0 % Calcium, optional mehr als 0,0 %, insbesondere mehr als 0,05 %, bis 1,2 % Ytterbium, Rest Magnesium und herstellungsbedingte Verunreinigungen aufweist.
Diese Legierung weist ausgezeichnete Degradations- bzw. Resorptionseigenschaften im biologischen Milieu einer Bandscheibe auf, zeigt eine hohe Duktilität und hat sich als besonders robust, besonders im Hinblick auf eine Ausbildung des Erstreckungskörpers als expandierbarer Körper mit bzw. aus dieser Mg-Zn-Ca-Legierung erwiesen, welcher große Volumenunterschiede und/oder Geometrieunterschiede zwischen dem Positionierungszustand und Erstreckungszustand des expandierbaren Körpers gewährleisten soll. Darüber hinaus hat sich gezeigt, dass mit dieser Mg-Zn-Ca-Legierung bzw. -Legierungszusammensetzung die Möglichkeit gegeben ist, eine Degradationsgeschwindigkeit sehr genau einzustellen, indem die Legierungsanteile innerhalb der vorgenannten Bereiche variiert werden, ohne dabei eine vorteilhafte Robustheit bzw. Belastbarkeit und Festigkeit bei gleichzeitig hoher Duktilität zu unterminieren. Eine gute Einstellbarkeit der Eigenschaften der Legierung ist insbesondere gegeben, wenn vorgenannte Elementanteile in der Legierung jeweils einen minimalen Anteil von mehr als 0,1 Gew.-% aufweisen, wobei bevorzugt ein minimaler Anteil von Ytterbium mehr als 0,05 Gew.-% beträgt. Bevorzugt für eine besonders hohe Robustheit und Festigkeit ist es, wenn die Mg-Zn-Ca-Legierung (in Gew.-%) 0,4 % bis 1,2 % Zink und 0,3 % bis 0,7 % Ca aufweist. Ein hoher Degradationswiderstand, also eine niedrige Degradationsgeschwindigkeit, und eine hohe Festigkeit ist insbesondere erreichbar, wenn in den vorgenannten Mg-Zn-Ca-Legierungszusammensetzungen (in Gew.-%) ein Ca-Anteil größer als ein Zn-Anteil ist. Dies erklärt sich dadurch, dass in diesem Zusammensetzungsbereich ein hoher Anteil von Mg₂Ca-Ausscheidungsphasen in der Legierung erzeugbar ist. Der Anteil von Mg₂Ca-Ausscheidungsphasen kann durch eine nach einer Herstellung der Legierung durchgeführte Wärmebehandlung weiter erhöht werden. Günstig ist es, wenn in den vorgenannten Mg-Zn-Ca-Legierungszusammensetzungen (in Gew.-%) mehr als 0,0 %, insbesondere mehr als 0,05 %, bis 1,2 % Ytterbium vorhanden ist. Dadurch ist eine Heißrissneigung bei einem Erstarrungsvorgang im Rahmen der Herstellung der Mg-Zn-Ca-Legierung bzw. eines Bauteiles aus dieser reduzierbar, sodass sich die Mg-Zn-Ca-Legierung bzw. ein aus dieser hergestelltes Implantatteil, insbesondere der Erstreckungskörper, im Speziellen wenn dieser als expandierbarer Körper ausgebildet ist, durch eine besonders hohe Belastbarkeit, vor allem bei wiederholten Langzeitbelastungen, auszeichnet. Besonders günstig ist es hierfür, wenn (in Gew.-%) 0,05 % bis 0,8 %, bevorzugt 0,4 % bis 0,8 %, Ytterbium vorhanden sind. Indem das Implantat bzw. Implantatteile, insbesondere der Erstreckungskörper, im Speziellen der expandierbare Körper bzw. der Basiskörper und/oder der Blockierkörper, und/oder die Mediumzufuhrleitung und/oder das Durchflussventil und/oder die Rückschlageinrichtung, mit bzw. aus einer Mg-Zn-Ca-Legierung gemäß vorgenannter Zusammensetzung ausgebildet sind, können diese somit entsprechend vorgenannter Wirkungen besonders an die Belastungsverhältnisse in einer Bandscheibe bzw. deren Bandscheibenzwischenwirbelraum angepasst werden. Dies gilt im besonderen Maße, wenn der expandierbare Körper mit, insbesondere aus, einer solchen Mg-Zn-Ca-Legierung gebildet ist, da dieser aufgrund der besonderen Kombination von Festigkeit und Duktilität vorgenannter Legierungszusammensetzung besonders robust ausgebildet werden kann, um in dessen Erstreckungszustand den hohen Belastungen an einer Einsatzposition in der Bandscheibe bzw. deren Bandscheibenzwischenwirbelraum nachhaltig standzuhalten, ohne dass dabei Abstriche hinsichtlich einer Biodegradierbarkeit in Kauf genommen werden müssen.

Vorteilhaft ist es, wenn eine Degradationsrate des Implantates bzw. einzelner Teile des Implantates gezielt eingestellt ist, um einen Heilungs- oder Regenerationsprozess der Bandscheibe bzw. des Bandscheibenzwischenwirbelraumes zu optimieren. Hierzu hat es sich bewährt, wenn an einer Einsatzposition bzw. einem Einsatzort des Implantates die Mediumzufuhrleitung bzw. deren bioresorbierbares Material und/oder gegebenenfalls das Durchflussventil bzw. dessen bioresorbierbares Material und/oder gegebenenfalls die Rückschlageinrichtung bzw. deren bioresorbierbares Material eine höhere Degradationsgeschwindigkeit als der Erstreckungskörper, insbesondere der expandierbare Körper bzw. der Basiskörper und/oder Blockierkörper, bzw. dessen bioresorbierbare Mg-Zn-Ca-Legierung aufweist. Bevorzugt ist dabei, dass die Mediumzufuhrleitung bzw. deren bioresorbierbares Material eine höhere Degradationsgeschwindigkeit als der Erstreckungskörper, insbesondere der expandierbare Körper bzw. der Basiskörper und/oder Blockierkörper, bzw. dessen bioresorbierbare Mg-Zn-Ca-Legierung aufweist. Auf diese Weise ist ermöglicht, sicherzustellen, dass sich an der Einsatzposition des Implantates zuerst die Mediumzufuhrleitung bzw. das Durchflussventil bzw. die Rückschlageinrichtung auflöst und erst anschließend der Erstreckungskörper, insbesondere der expandierbare Körper bzw. Basiskörper und/oder Blockierkörper, dessen strukturelle Integrität verliert. Dieser sukzessive Verlust der strukturellen Integrität des Implantates erlaubt eine gezielte Abstimmung auf den Heilungsprozess der Bandscheibe. Bevorzugt ist es zusätzlich, wenn am Einsatzort bzw. der Einsatzposition des Implantates die Mediumzufuhrleitung bzw. deren bioresorbierbares Material eine höhere Degradationsgeschwindigkeit als das Durchflussventil bzw. dessen bioresorbierbares Material und/oder gegebenenfalls die Rückschlageinrichtung bzw. deren bioresorbierbares Material aufweist. Auf diese Weise kann eine Abnahme bzw. ein Verlust der strukturellen Integrität des Implantates besonders gut auf den Heilungsprozess der Bandscheibe abgestimmt werden. Um das Degradationsverhalten gezielt einzustellen, ist es günstig, wenn sowohl die Mediumzufuhrleitung als auch gegebenenfalls das Durchflussventil und gegebenenfalls die Rückschlageinrichtung mit einer Mg-Zn-Ca-Legierung ausgebildet sind. Besonders präzise ist das Degradationsverhalten des Implantates einstellbar, wenn der Erstreckungskörper, insbesondere der expandierbare Körper bzw. der Basiskörper bzw. der Blockierkörper, die Mediumzufuhrleitung und gegebenenfalls das Durchflussventil und gegebenenfalls die Rückschlageinrichtung mit bzw. aus einer Mg-Zn-Ca-Legierung gemäß einer vorgenannten Zusammensetzung, insbesondere mit den entsprechenden vorgenannten Wirkungen, ausgebildet sind. Es versteht sich, dass der Erstreckungskörper, insbesondere der expandierbare Körper bzw. der Basiskörper und/oder der Blockierkörper, die Mediumzufuhrleitung und gegebenenfalls das Durchflussventil und gegebenenfalls die Rückschlageinrichtung dabei in der Regel mit bzw. aus jeweils einer unterschiedlichen Mg-Zn-Ca-Legierungszusammensetzung gemäß vorgenannter Legierungsdefinitionen ausgebildet sein können, um das gewünschte Degradationsverhalten einzustellen. Um eine Tragfähigkeit des Erstreckungskörpers auf einen konkreten Anwendungsfall abzustimmen, kann es vorteilhaft sein, wenn der Erstreckungskörper teilweise aus einer, insbesondere vorgenannten, bioresorbierbaren Mg-Zn-Ca-Legierung und teilweise aus einem anderen biokompatiblen, insbesondere bioresorbierbaren, Material, wie beispielsweise einem Polymer, Titan, Polylactid oder einem Derivat davon und/oder Schweinedarm gebildet ist.

Als günstig hat es sich herausgestellt, wenn der Erstreckungskörper, im Speziellen der expandierbare Körper bzw. der Basiskörper und/oder der Blockierkörper, mit bzw. aus zumindest einem mit der bioresorbierbaren Mg-Zn-Ca-Legierung gebildeten Draht, insbesondere Drahtgeflecht oder Drahtgewebe, ausgebildet ist. Dies ermöglicht eine einfache und praktikable Herstellung und insbesondere infolge eine hohe gleichbleibende Qualität, um insbesondere den speziellen Anforderungen an eine Belastbarkeit in einer Bandscheibe gerecht zu werden. Bevorzugt ist der Erstreckungskörper, im Speziellen der expandierbare Körper bzw. Basiskörper und/oder Blockierkörper, hierzu mit bzw. aus mehreren solchen Drähten, welche insbesondere miteinander, im Besonderen in Form eines Geflechtes, Gewebes oder Netzes, verbunden sind, gebildet. Dadurch ist der Erstreckungskörper mit einem hohen Tragfähigkeits-Gewichtsverhältnis ausbildbar. Insbesondere kann dadurch der expandierbare Körper mit einer besonders ausgeprägten Expansionsfähigkeit, also einem großen Unterschied in dessen Volumen zwischen dessen Positionierungszustand und dessen Erstreckungszustand, umgesetzt werden, insbesondere wenn der expandierbare Körper ballonartig, kissenartig oder schlauchartig ausgebildet ist. Je nach Einsatzzweck kann es dabei günstig sein, wenn der Erstreckungskörper bzw. der expandierbare Körper sowohl mit Drähten gebildet ist, welche mit bzw. aus der bioresobierbaren Mg-Zn-Ca-Legierung gebildet sind, als auch mit Drähten gebildet ist, welche mit bzw. aus einem anderen biokompatiblen, insbesondere bioresorbierbaren, Material, wie beispielsweise Polylactid oder einem Derivat davon gebildet sind. Auf diese Weise kann eine Tragfähigkeit des Erstreckungskörpers spezifisch auf einen konkreten Einsatzzweck abgestimmt eingestellt werden. Im Besonderen gilt Vorgenanntes in analoger Weise für den Basiskörper und/oder Blockierkörper.

Eine einfache Handhabung ist umsetzbar, wenn der Erstreckungskörper, im Speziellen der expandierbare Körper, in dessen Positionierungszustand eine Längserstreckung aufweist, welche mindestens doppelt, insbesondere mindestens dreimal, bevorzugt mindestens viermal, so groß wie eine Quererstreckung des Erstreckungskörpers bzw. expandierbaren Körpers in dessen Positionierungszustand ist. Damit ist eine längliche Form des Erstreckungskörpers bzw. expandierbaren Körpers erreichbar, welche ein einfaches Einfügen des Erstreckungskörpers bzw. expandierbaren Körpers in die Bandscheibe durch eine schmale Öffnung bzw. ein kleines Bohrloch in der Bandscheibe, insbesondere dem Anulus-Fibrosus, ermöglicht. Bewährt hat es sich dabei, wenn eine, insbesondere maximale, Quererstreckung des Erstreckungskörpers bzw. expandierbaren Körpers in dessen Positionierungszustand zwischen 2 mm und 20 mm, insbesondere zwischen 2 mm und 10 mm, bevorzugt zwischen 2 mm und 8 mm, besonders bevorzugt zwischen 2 mm und 5 mm, aufweist.

Längserstreckung des Erstreckungskörpers bzw. expandierbaren Körpers bezeichnet eine Erstreckung, demnach eine Länge, des Erstreckungskörpers bzw. expandierbaren Körpers in dessen Längsrichtung, also in Richtung dessen längster Erstreckung. Quererstreckung des Erstreckungskörpers bzw. expandierbaren Körpers bezeichnet eine Erstreckung, demnach eine Länge, des Erstreckungskörpers bzw. expandierbaren Körpers in einer Richtung orthogonal bzw. rechtwinklig zur Längsrichtung des Erstreckungskörpers bzw. expandierbaren Körpers.

Günstig für ein schonendes Einsetzen des Implantates ist es, wenn eine Quererstreckung des Erstreckungskörpers bzw. expandierbaren Körpers in dessen Erstreckungszustand mindestens doppelt, insbesondere mindestens dreimal, bevorzugt mindestens viermal, so groß wie eine Quererstreckung des Erstreckungskörpers in dessen Positionierungszustand ist. Insbesondere in Kombination mit vorgenannten Verhältnissen von Längserstreckung zu Quererstreckung im Positionierungszustand des Erstreckungskörpers ist dadurch ermöglicht, den Erstreckungskörper durch eine kleine Öffnung im Anulus-Fibrosus in die Bandscheibe einzubringen und anschließend auf eine wirkungsvolle Ausdehnung des Erstreckungskörpers zur Wiederherstellung von gewünschten Druckverhältnissen in der Bandscheibe zu expandieren. Hierzu ist es besonders günstig, wenn der Erstreckungskörper in dessen Positionierungszustand eine Längserstreckung aufweist, welche mindestens dreimal so groß wie eine Quererstreckung des Erstreckungskörpers in dessen Positionierungszustand ist und eine Quererstreckung des Erstreckungskörpers in dessen Erstreckungszustand mindestens doppelt, bevorzugt mindestens dreimal so groß wie eine Quererstreckung des Erstreckungskörpers in dessen Positionierungszustand ist. Um einem intradiskalen Druck effizient und nachhaltig standzuhalten ist es meist günstig, wenn der Erstreckungskörper in dessen Erstreckungszustand an einer Einsatzposition des Erstreckungskörpers in der Bandscheibe in einer Richtung orthogonal zu einer Deckplatte eines der Bandscheibe benachbarten Wirbelkörpers eine Höhe aufweist, welche in etwa einer Höhe der zu behandelnden Bandscheibe entspricht. Je nach zu behandelnder Bandscheibe ist es hierzu zweckmäßig, wenn die Höhe des Erstreckungskörpers in dessen Erstreckungszustand zwischen 2 mm und 8 mm, bevorzugt zwischen 3 mm und 7 mm, häufig etwa 5 mm, beträgt. Es versteht sich, dass vorstehend genannte Ausbildungen und Abmessungen des Erstreckungskörpers im Besonderen auch für einen expandierbaren Körper gelten, mit welchem bzw. als welcher der Erstreckungskörpers vorzugsweise ausgebildet sein kann.

Vorteilhaft ist es, wenn der Erstreckungskörper bzw. expandierbare Körper in dessen Erstreckungszustand an gegenüberliegenden Seiten des Erstreckungskörpers bzw. expandierbaren Körpers im Wesentlichen parallel zueinander angeordnete, insbesondere ebene, Außenflächen aufweist, um in einer Einsatzposition des Implantates mit den Außenflächen eine Druckkraft auf Deckplatten von an die Bandscheibe angrenzenden Wirbeln bzw. Wirbelkörpern aufzubringen. Dadurch ist eine effiziente Kraftübertragung, insbesondere zur Erzeugung eines gewünschten Wirbelkörperabstandes, ermöglicht.

Eine besonders gute Stützfunktionalität des Implantates ist erreichbar, wenn der Erstreckungskörper bzw. expandierbare Körper in dessen Erstreckungszustand eine Form eines Schlauches, dessen Enden miteinander verbunden sind, insbesondere eine Form eines Torus, aufweist.

Es hat sich bewährt, dass der Erstreckungskörper bzw. expandierbare Körper eine schlauchförmige Ausstülpung aufweist, welche eine vorgenannte Füllöffnung, insbesondere mehrere vorgenannte Füllöffnungen, des expandierbaren Körpers bildet. Die Ausstülpung ist vorzugsweise derart ausgebildet, dass bei einer zumindest teilweisen Anordnung, üblicherweise in Form eines Einführens, des Erstreckungskörpers bzw. expandierbaren Körpers durch das Bohrloch oder die Öffnung im Anulus-Fibrosus in den Hohlraum der Bandscheibe die Ausstülpung im, bevorzugt durch das, Bohrloch bzw. in der, bevorzugt durch die, Öffnung des Anulus-Fibrosus von innerhalb der Bandscheibe nach außen gerichtet ist bzw. nach außen ragt. Dadurch wird eine einfache Positionierung des Implantates bzw. Erstreckungskörpers ermöglicht. Im Besonderen ermöglicht dies ein einfaches und leicht zu handhabendes Befüllen des Hohlraumes bzw. expandierbaren Körpers mit Füllmedium. Je nach konkreter Ausgestaltung kann die Ausstülpung dabei über einen Außenfläche des Anulus-Fibrosus nach außen gerichtet überstehen oder plan mit der Außenfläche abschließen oder innerhalb der Bandscheibe bzw. des Anulus-Fibrosus bzw. dessen Öffnung oder Bohrloch enden. Es versteht sich, dass es üblicherweise zweckmäßig ist, wenn die Ausstülpung orthogonal zur Längsachse der Ausstülpung eine maximale Breite bzw. eine maximale Querschnittsfläche aufweist, welche kleiner ist als eine maximale Breite bzw. maximale Querschittsfläche des Erstreckungskörpers, im Speziellen des expandierbaren Körpers, in dessen Erstreckungszustand orthogonal zu dieser Längsachse. Indem die Ausstülpung zumindest teilweise im Bohrloch oder in der Öffnung des Anulus-Fibrosus angeordnet ist, ist außerdem ein praktikables zumindest teilweises, insbesondere gänzliches, Verschließen des Bohrloches bzw. der Öffnung im Anulus-Fibrosus ermöglicht. Auf diese Weise ist insbesondere ein Austritt von Nucleus-Pulposus-Material durch das Bohrloch bzw. die Öffnung im Anulus-Fibrosus verhinderbar. Zweckmäßig ist es, wenn die schlauchförmige Ausstülpung expandierbar ausgebildet ist, sodass ein Befüllen der Ausstülpung mit Füllmedium einen Querschnitt der Ausstülpung vergrößert. Dadurch kann die Ausstülpung als Art Pfropfen wirken, welche das Bohrloch bzw. die Öffnung im Anulus-Fibrosus verschließt. Mit dieser Umsetzung ist es ermöglicht, die Öffnung bzw. das Bohrloch im Anulus-Fibrosus nachhaltig zu verschließen, insbesondere ohne dass ein zusätzliches separates Anulus-Fibrosus-Verschlusssystem, beispielsweise ein Verkleben der Öffnung bzw. des Bohrloches im Anulus-Fibrosus erforderlich ist. Es versteht sich, dass es zweckdienlich sein kann, wenn der Erstreckungskörper bzw. expandierbare Körper mehrere vorgenannte schlauchförmige Ausstülpungen aufweist und im Besonderen der Erstreckungskörper bzw. expandierbare Körper derart im Hohlraum der Bandscheibe anordenbar ist, dass die Ausstülpungen zumindest teilweise durch unterschiedliche Bohrlöcher oder Öffnungen im Anulus-Fibrosus von innerhalb der Bandscheibe nach außen gerichtet sind bzw. nach außen ragen.

Vorteilhaft ist es, wenn die Mediumzufuhrleitung mit der Ausstülpung des expandierbaren Körpers verbindbar bzw. verbunden ist, sodass Füllmedium über die mit der Ausstülpung gebildete Füllöffnung dem Hohlraum der Bandscheibe bzw. einem oder mehreren Füllräumen des expandierbaren Körpers zuführbar ist. Wie vorstehend bereits ausgeführt, ist es zweckmäßig, wenn die Mediumzufuhrleitung mit der Ausstülpung des Erstreckungskörpers bzw. expandierbaren Körpers formschlüssig oder kraftschlüssig - bevorzugt mit einem Schnellverschluss - insbesondere lösbar, verbindbar bzw. verbunden ist. Bevorzugt ist es dabei, entsprechend vorstehenden Ausführungen, wenn ein Randbereich der Füllöffnung des Erstreckungskörpers bzw. expandierbaren Körpers bzw. dessen Ausstülpung über einen Abschnitt der Mediumzufuhrleitung stülpbar und insbesondere kraftschlüssig, beispielsweise mit dem Klemmelement, insbesondere Klemmring, mit der Mediumzufuhrleitung verbindbar ist. Üblicherweise weist die Mediumzufuhrleitung einen Querschnitt auf, welcher kleiner ist als ein Querschnitt der Ausstülpung. Es kann aber auch günstig sein, wenn die Mediumzufuhrleitung zumindest einen Querschnitt aufweist, welcher größer ist als ein Querschnitt der Ausstülpung, sodass die Mediumzufuhrleitung in einem mit der Ausstülpung verbundenen Zustand nicht gänzlich durch die Öffnung bzw. das Bohrloch im Anulus-Fibrosus durchführbar ist. Auf diese Weise ist erreichbar, dass die Mediumzufuhrleitung in einer Einsatzposition des Implantates an einer Außenseite der Bandscheibe einen Anschlag bildet, welcher ein, insbesondere gänzliches, Herausrutschen der Ausstülpung aus der Öffnung bzw. dem Bohrloch im Anulus-Fibrosus in Richtung eines Bandscheibeninneren bzw. in Einschubrichtung des Implantates unterbindet und insbesondere eine formschlüssige Verbindung des Implantates mit dem Anulus-Fibrosus der Bandscheibe umsetzt. Im Besonderen ist es dadurch ermöglicht, große und/oder nicht-kreisförmige, insbesondere etwa schlitzförmige Bohrlöcher bzw. Öffnungen, etwa Risse, im Anulus-Fibrosus zu verschließen.

Für eine Positionierung des Implantates ist es praktikabel, wenn die Mediumzufuhrleitung ein Segment aufweist, welches mit einer Mehrkantoberfläche ausgebildet ist, um ein Festhalten und Bewegen der Mediumzufuhrleitung mit einem Werkzeug, beispielsweise einem Gabelschlüssel, zu ermöglichen. Günstig ist es, wenn die Mediumzufuhrleitung eine oder mehrere Sollbruchstellen aufweist, sodass das Segment mit der Mehrkantoberfläche von der Mediumzufuhrleitung durch Brechen der Sollbruchstellen abtrennbar ist. Damit kann das Segment mit der Mehrkantoberfläche, welches lediglich für die Positionierung des Implantates funktionell ist, nach der Positionierung bzw. nach Überführen des Erstreckungskörpers bzw. expandierbaren Körpers in den Erstreckungszustand vom Implantat entfernt werden, wodurch eine durch das Implantat gebildete Fremdkörpermenge im biologischen System reduzierbar ist.

Um einen Austritt von Füllmedium aus dem Implantat zu verhindern, ist es günstig, wenn das Implantat eine Verschließeinrichtung aufweist, um die Mediumzufuhrleitung und/oder die Füllöffnung des Implantates bzw. des Erstreckungskörpers, insbesondere des expandierbaren Körpers, für einen Durchgang von Füllmedium zu verschließen. Dies kann auf einfache Weise umgesetzt sein, indem die Verschließeinrichtung ein Verschließelement umfasst, welches, bevorzugt unter Bildung einer formschlüssigen und/oder kraftschlüssigen Verbindung, in die Mediumzufuhrleitung und/oder den Erstreckungskörper bzw. expandierbaren Körper, insbesondere die Ausstülpung des Erstreckungskörpers bzw. expandierbaren Körpers, einfügbar ist, sodass die Mediumzufuhrleitung und/oder die Füllöffnung des Erstreckungskörpers bzw. expandierbaren Körpers für einen Durchgang von Füllmedium verschlossen ist. Hierzu kann beispielsweise die Verschließeinrichtung mit einer Verschließschraube gebildet sein, welche in ein zur Verschließschraube korrespondierendes Gewinde der Mediumzufuhrleitung und/oder des Erstreckungskörpers bzw. expandierbaren Körpers einschraubbar ist, um die Mediumzufuhrleitung und/oder die Füllöffnung des Erstreckungskörpers bzw. expandierbaren Körpers für einen Durchgang von Füllmedium zu verschließen. Für eine hohe Biokompatibilität ist es günstig, wenn die Verschließeinrichtung, insbesondere die Verschließschraube, mit bzw. aus einem bioresorbierbaren Material, bevorzugt einer vorgenannten Mg-Zn-Ca-Legierung, gebildet ist.

Mit Vorteil kann vorgesehen sein, dass der Erstreckungskörper, insbesondere expandierbare Körper bzw. Basiskörper und/oder Blockierkörper, mehrere Bereiche aufweist, welche mit bzw. aus unterschiedlichen biodegradierbaren Materialien, insbesondere biodegradierbaren Mg-Zn-Ca-Legierungen, mit unterschiedlichen Degradationsgeschwindigkeiten gebildet sind. Auf diese Weise ist der Erstreckungskörper auf einen Heilungsprozess bzw. Regenerationsprozess der Bandscheibe abstimmbar, da der Verlust einer strukturellen Integrität des Erstreckungskörpers gezielt einstellbar ist. Für eine nachhaltige Robustheit und Stabilität ist es zweckmäßig, wenn die Bereiche mit, bevorzugt aus, unterschiedlichen biodegradierbaren Mg-Zn-Ca-Legierungen gemäß einer vorgenannten Mg-Zn-Ca-Legierungszusammensetzung gebildet sind.

Das weitere Ziel der Erfindung wird durch ein Verfahren der eingangs genannten Art zur Herstellung eines Implantates erreicht, bei welchem das Implantat bzw. ein Teil des Implantates, insbesondere der Erstreckungskörper, im Speziellen der expandierbare Körper bzw. Basiskörper und/oder Blockierkörper, und/oder die Mediumzufuhrleitung und/oder gegebenenfalls die Rückschlageinrichtung und/oder gegebenenfalls die Verschließeinrichtung, mit einem additiven Fertigungsverfahren, bevorzugt einem 3-D-Druck-Verfahren, hergestellt ist. Es hat sich gezeigt, dass biodegradierbare Mg-Zn-Ca-Legierungen für eine Verarbeitung mit additiven Fertigungsverfahren, im Besonderen 3-D-Druckverfahren, geeignet sind. Dadurch ist es ermöglicht, auch komplexe Formen herzustellen, welche auf einen spezifischen Behandlungsfall optimiert und angepasst werden können. Insbesondere ist es durch einen schichtweisen Herstellungsprozess relativ einfach ermöglicht, unterschiedliche Bereiche bzw. Abschnitte eines Implantatteiles mit unterschiedlichen Material- bzw. Legierungszusammensetzungen auszubilden. Bevorzugt ist es im Besonderen, wenn der Erstreckungskörper bzw. expandierbare Körper mit einem solchen Verfahren hergestellt wird.

Der Begriff 3-D-Druck-Verfahren umfasst dabei die dem Fachmann bekannten zugehörigen Verfahren, insbesondere die Verfahren Multi-Jet-Modeling, Fused-Deposition-Modeling, selektives Lasersintern sowie Elektronenstrahlsintern.

Alternativ kann es günstig sein, wenn das Implantat bzw. ein Teil des Implantates mit einem Gießverfahren und/oder Strangpressen und/oder spanenden Fertigungsverfahren und/oder mit Drahtziehen hergestellt wird.

Auf praktikable Weise ist somit ein Verfahren zur Behandlung einer Bandscheibe mit einem Implantat durchführbar, indem das Implantat als erfindungsgemäßes Implantat ausgebildet ist, wobei zumindest ein Teil der Bandscheibe durch zumindest einen Teil des Implantates ersetzt wird und/oder zumindest ein Teil des Implantates in die Bandscheibe eingeführt wird. Vorteilhaft ist dabei vorgesehen, dass der Erstreckungskörper, im Speziellen der expandierbare Körper bzw. Basiskörper und/oder Blockierkörper, in dessen Positionierungszustand zumindest teilweise in einem Bandscheibenzwischenwirbelraum der Bandscheibe positioniert und der Erstreckungskörper, insbesondere expandierbare Körper, anschließend in dessen Erstreckungszustand überführt, insbesondere expandiert, wird. Entsprechend den vorstehend erläuterten Ausführungen, Merkmalen und Wirkungen eines erfindungsgemäßen Implantates ist durch die Behandlung einer Bandscheibe mit einem solchen eine hohe Belastbarkeit der behandelten Bandscheibe und verbesserte Heilung bzw. Regeneration der Bandscheibe erreichbar.

Üblicherweise wird dies praktikabel umgesetzt, indem der Erstreckungskörper bzw. expandierbare Körper in dessen Positionierungszustand zumindest teilweise durch eine Öffnung in einem Anulus-Fibrosus der Bandscheibe in einen Hohlraum der Bandscheibe eingefügt wird, wonach der Erstreckungskörper, insbesondere expandierbare Körper, in dessen Erstreckungszustand überführt, insbesondere expandiert, wird, um mit dem Erstreckungskörper bzw. expandierbaren Körper einen Nucleus-Pulposus der Bandscheibe zumindest teilweise zu ersetzen und/oder die Öffnung im Anulus-Fibrosus zu verschließen.

Es versteht sich, dass das Verfahren zur Herstellung eines Implantates bzw. das Verfahren zur Behandlung einer Bandscheibe entsprechend bzw. analog den Merkmalen, Vorteilen und Wirkungen, welche im Rahmen eines erfindungsgemäßen Implantates, insbesondere vorstehend, beschrieben sind, ausgeführt werden kann bzw. ausgebildet sein kann. Analoges gilt auch für das erfindungsgemäße Implantat im Hinblick auf die Verfahren.

Weitere Merkmale, Vorteile und Wirkungen ergeben sich aus den nachfolgend dargestellten Ausführungsbeispielen. In den Zeichnungen, auf welche dabei Bezug genommen wird, zeigen:
Fig. 1 eine schematische Darstellung eines erfindungsgemäßen Implantates mit einem Erstreckungskörper in dessen Erstreckungszustand sowie eine Bandscheibe in einem Querschnitt der Bandscheibe;
Fig. 2 eine schematische Darstellung des Implantates der Fig. 1 mit dem Erstreckungskörper in dessen Positionierungszustand;
Fig. 3 eine schematische Darstellung eines weiteren erfindungsgemäßen Implantates mit einem Erstreckungskörper in dessen Erstreckungszustand;
Fig. 4 eine schematische Explosionsdarstellung eines Ausschnittes der Implantate der Fig. 1 bis Fig. 3;
Fig. 5 eine schematische Darstellung eines weiteren erfindungsgemäßen Implantates mit einem Erstreckungskörper in dessen Erstreckungszustand;
Fig. 6 eine schematische Darstellung der Mediumzufuhrleitung samt Durchflussventil der Fig. 5;
Fig. 7 eine schematische Darstellung eines weiteren erfindungsgemäßen Implantates mit einem Erstreckungskörper in dessen Erstreckungszustand eingefügt in einer Bandscheibe;
Fig. 8 eine schematische Darstellung des Implantates der Fig. 7 mit einem Erstreckungskörper in dessen Positionierungszustand;
Fig. 9 eine schematische Darstellung des Implantates der Fig. 7 und Fig. 8 mit dem Erstreckungskörper in dessen Erstreckungszustand.

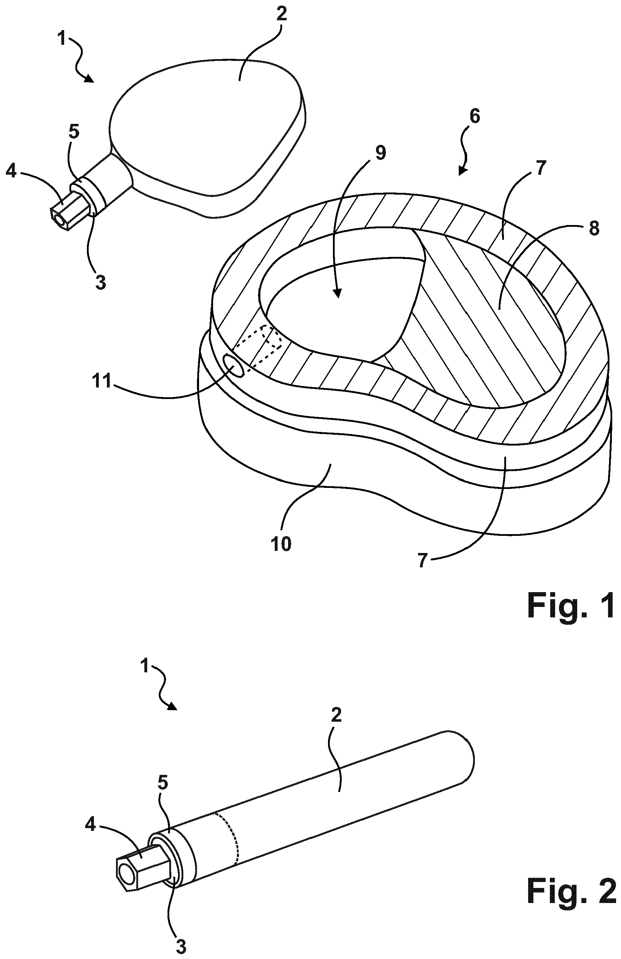

Fig. 1 zeigt eine schematische Darstellung einer Ausführungsvariante eines Implantates 1 und eine schematische Darstellung einer Bandscheibe 6 in einem Querschnitt durch die Bandscheibe 6. Das Implantat 1 weist einen Erstreckungskörper, welcher als expandierbarer Körper 2 ausgebildet ist, und eine mit diesem verbundene Mediumzufuhrleitung 3 auf. Der expandierbare Körper 2 ist ausgebildet, von einem Positionierungszustand in einen Erstreckungszustand, in welchem der expandierbare Körper 2 ein größeres Volumen aufweist als im Positionierungszustand, expandiert zu werden. Hierfür weist der expandierbare Körper 2 eine ballonartige Gestalt auf, mit welcher ein mit einem Füllmedium befüllbarer Füllraum gebildet ist, um den expandierbaren Körper 2 durch Befüllen des Füllraumes mit Füllmedium über die Mediumzufuhrleitung 3 von dessen Positionierungszustand in dessen Erstreckungszustand zu expandieren. In Fig. 1 ist der Erstreckungskörper bzw. expandierbare Körper 2 in dessen Erstreckungszustand dargestellt.

Weiter ist in Fig. 1 eine schematische Darstellung einer Bandscheibe 6 in einem Querschnitt durch die Bandscheibe 6 mit darunter angeordnetem Wirbelkörper 10 dargestellt, um eine übliche Positionierung des Implantates 1 in der Bandscheibe 6 bei einer Behandlung der Bandscheibe 6 zu veranschaulichen. Ersichtlich sind ein Anulus-Fibrosus 7 sowie ein Nucleus-Pulposus 8 der Bandscheibe 6, wobei der Nucleus-Pulposus 8 vom Anulus-Fibrosus 7 etwa ringartig umfasst ist. Dargestellt ist ein Behandlungszustand, in welchem ein Bohrloch 11 in den Anulus-Fibrosus 7 eingebracht und ein Teil des Nucleus-Pulposus 8 entfernt ist, sodass ein Hohlraum 9 in der Bandscheibe 6 gebildet ist. In einem üblichen Verfahren zur Behandlung der Bandscheibe 6 wird der expandierbare Körper 2 in dessen Positionierungszustand durch das Bohrloch 11 zumindest teilweise in den Hohlraum 9 der Bandscheibe 6 eingebracht bzw. eingeführt, wonach der expandierbare Körper 2 in dessen Erstreckungszustand expandiert wird, indem über die Mediumzufuhrleitung 3 bzw. eine Füllöffnung des expandierbaren Körpers 2 ein Füllmedium in den Füllraum des expandierbaren Körpers 2 eingebracht wird. Es versteht sich, dass in der Darstellung der Fig. 1 das dargestellte Implantat 1 im Sinne einer Explosionsdarstellung nur zur besseren Veranschaulichung aus der Bandscheibe 6 enthoben dargestellt ist und in einer Einsatzposition des Implantates in der Bandscheibe tatsächlich der expandierbare Körper 2 des Implantates 1 zumindest teilweise oder gänzlich im Hohlraum 9 der Bandscheibe 6 angeordnet ist, wobei die Mediumzufuhrleitung 3 durch das Bohrloch 11 von innerhalb der Bandscheibe nach außen ragt und insbesondere zumindest teilweise, häufig gänzlich, über eine Außenfläche der Bandscheibe bzw. des Anulus-Fibrosus übersteht. Je nach konkretem Einsatzzweck kann es aber günstig sein, wenn die Mediumzufuhrleitung gänzlich innerhalb der Bandscheibe angeordnet wird, insbesondere derart, dass die Mediumzufuhrleitung gänzlich in einem Bereich eines Nucleus-Pulposus der Bandscheibe angeordnet ist bzw. nicht in das Bohrloch bzw. eine Öffnung des Anulus-Fibrosus ragt.

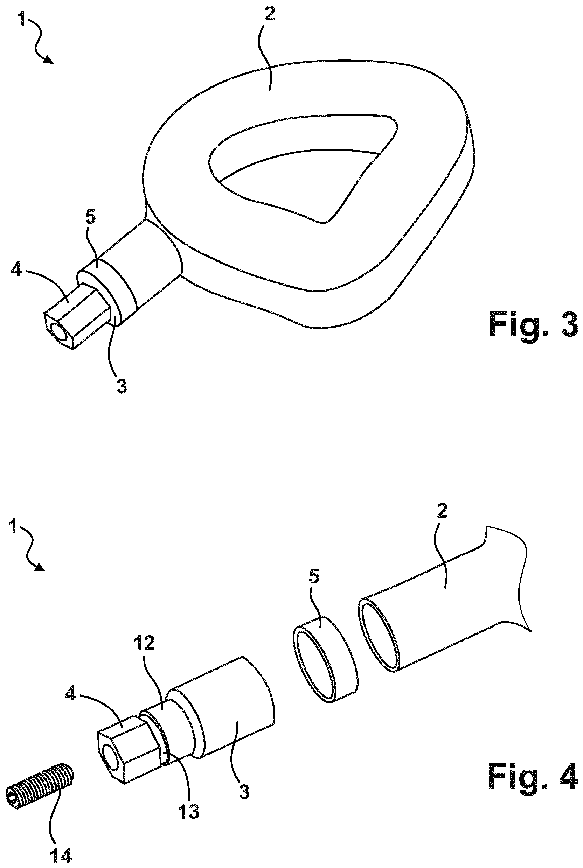

Der expandierbare Körper 2 ist mit bzw. aus einer bioresorbierbaren Mg-Zn-Ca-Legierung gebildet, wodurch eine hohe Belastbarkeit und hohe Biokompatibilität erreichbar ist. Dabei kann es günstig sein, wenn der expandierbare Körper teilweise aus einer bioresorbierbaren Mg-Zn-Ca-Legierung und teilweise aus einem zumindest biokompatiblen Material, insbesondere einem anderen bioresorbierbarem Material, beispielsweise Polylactid oder einem Derivat davon, gebildet ist. Die Mediumzufuhrleitung 3 ist bevorzugt mit bzw. aus einem rohrförmigen, starren Bauteil ausgebildet, wodurch eine Handhabung und Positionierung des Implantates 1 erleichtert ist. Eine belastbare Verbindung zwischen der Mediumzufuhrleitung 3 und dem expandierbaren Körper 2 ist umgesetzt, indem eine schlauchförmige Ausstülpung des expandierbaren Körpers 2, welche eine Füllöffnung des expandierbaren Körpers bildet, über einen Abschnitt der Mediumzufuhrleitung 3 gestülpt ist und bevorzugt, zweckmäßigerweise mit einem Klemmring 5, kraftschlüssig, in der Regel unter Bildung einer Klemmverbindung, mit der Mediumzufuhrleitung 3 verbunden ist; detailliert dargestellt in Fig. 4. Die Mediumzufuhrleitung 3 weist ein Segment mit einer Mehrkantoberfläche 4 auf, um ein Festhalten und Bewegen des Implantates 1 mit einem Werkzeug auf einfache Weise zu ermöglichen. Überlicherweise weist das Implantat 1 außerdem eine Verschließschraube 14 auf, welche in ein Gewinde der Mediumzufuhrleitung 3 einschraubbar ist, um die Mediumzufuhrleitung 3 für einen Durchgang von Füllmedium durch die Mediumzufuhrleitung 3 zu verschließen.

Fig. 2 zeigt eine schematische Darstellung des Implantates 1 der Fig. 1, wobei das Implantat 1 bzw. der expandierbare Körper 2 in dessen Positionierungszustand dargestellt ist. Der expandierbare Körper 2 weist in dessen Positionierungszustand eine hohlzylindrische Gestalt auf, wodurch das Implantat 1 bzw. der expandierbare Körper 2 auf einfache Weise zumindest teilweise durch das Bohrloch 11 im Anulus-Fibrosus 7 der Bandscheibe 6 in den Hohlraum 9 der Bandscheibe 6 einfügbar und erst nach Positionierung des expandierbaren Körpers 2 im Hohlraum 9 der Bandscheibe 6 durch Einfüllen eines Füllmediums in den expandierbaren Körper 2 in den Erstreckungszustand überführbar ist.

Fig. 3 zeigt eine schematische Darstellung einer weiteren Ausführungsvariante eines Implantates 1. Das Implantat 1 ist üblicherweise in gleicher Weise mit den gleichen Merkmalen und Wirkungen wie das Implantat 1 der Fig. 1 und Fig. 2 aufgebaut. Im Unterschied zum Implantat 1 der Fig. 1 und Fig. 2 ist der expandierbare Körper 2 mit einer Form eines Schlauches ausgebildet, dessen Enden miteinander verbunden sind. Fig. 3 zeigt das Implantat 1 in einem Erstreckungszustand des Erstreckungskörpers bzw. expandierbaren Körpers 2, in welchem das Implantat 1 eine torusähnliche Gestalt aufweist. In einem Positionierungszustand des expandierbaren Körpers 2 des Implantates 1 der Fig. 3 weist der expandierbare Körper 2 bevorzugt eine zur in Fig. 2 gezeigte analoge stabähnliche Gestalt auf.

Fig. 4 zeigt eine schematische Explosionsdarstellung der Implantate 1 der Fig. 1 bis Fig. 3, wobei ein Ausschnitt des Übergangsbereiches zwischen Mediumzufuhrleitung 3 und expandierbarem Körper dargestellt ist. Ersichtlich ist die Ausstülpung des expandierbaren Körpers 2, welche über einen Abschnitt der Mediumzufuhrleitung 3 stülpbar ist, um mit dem Klemmring 5 mit dieser unter Bildung einer Pressverbindung verbunden zu werden. Günstig ist es hierbei, dass die Mediumzufuhrleitung 3 eine zum Klemmring 5 korrespondierende Klemmringaufnahme 12 aufweist, in welche der Klemmring 5 zur Herstellung einer Pressverbindung formschlüssig einfügbar bzw. einpressbar ist. Zweckmäßig ist die Klemmringaufnahme 12 als Vertiefung in einer Außenfläche der Mediumzufuhrleitung 3 umsetzbar, wie ersichtlich in Fig. 4. Um das Segment der Mediumzufuhrleitung 3 mit der Mehrkantoberfläche 4 nach Positionierung des Implantates 1 bzw. Expandieren des expandierbaren Körpers 2 von der Mediumzufuhrleitung 3 abzutrennen, ist es zweckmäßig, wenn das Segment durch Sollbruchstellen 13 mit der Mediumzufuhrleitung 3 verbunden ist. Weiter in Fig. 4 ersichtlich ist eine Verschließschraube 14, welche in ein Gewinde der Mediumzufuhrleitung 3 einschraubbar ist, um die Mediumzufuhrleitung 3 für einen Durchgang von Füllmedium durch die Mediumzufuhrleitung 3 zu verschließen.

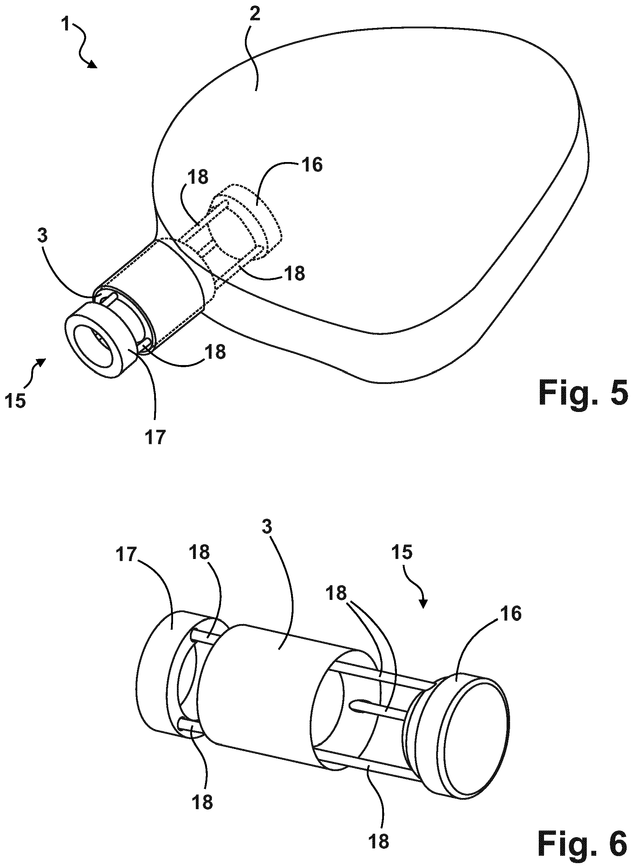

Fig. 5 zeigt eine weitere schematische Darstellung einer Ausführungsvariante eines Implantates 1. Der expandierbare Körper 2 des Implantates 1 ist in dessen Erstreckungszustand dargestellt und entspricht insbesondere dem expandierbaren Körper der Fig. 1. Das Implantat 1 der Fig. 5 kann mit den gleichen Merkmalen und Wirkungen wie die Implantate 1 der Fig. 1 bis Fig. 4 aufgebaut sein. Im Unterschied zu den Implantaten 1 der Fig. 1 bis Fig. 4 weist das Implantat 1 der Fig. 5 zusätzlich ein Durchflussventil 15 auf, um eine Durchflussmenge, insbesondere einen Volumenstrom, eines über die Füllöffnung des expandierbaren Körpers 2 dem Füllraum zugeführten Füllmediums zu steuern. Hierzu weist das Durchflussventil 15 ein Durchflussventilelement 16 und ein Durchflusssteuerelement 17 auf, welche mit Führungsstangen 18 verbunden sind, wobei die Führungsstangen 18 beweglich in Führungslagern der Mediumzufuhrleitung 3 gelagert sind, ersichtlich dargestellt auch in Fig. 6. Auf diese Weise ist durch Bewegen des Durchflusssteuerelementes 17 durch einen Bediener, beispielsweise einen Chirurgen, ein Durchflussquerschnitt der Mediumzufuhrleitung 3 in Abhängigkeit von einer Position des Durchflusssteuerelementes 17 änderbar, um eine Durchflussmenge, insbesondere einen Volumenstrom, von Füllmedium durch die Mediumzufuhrleitung 3 bzw. durch die Füllöffnung des expandierbaren Körpers 2 zu steuern.

Fig. 6 zeigt die Mediumzufuhrleitung 3 mit dem Durchflussventil 15 der Fig. 5 in einer anderen Ansicht. Ersichtlich ist, dass das Durchflusssteuerelement 17 als Verschlusspfropfen ausgebildet ist, um durch teilweises Verschließen der Mediumzufuhrleitung 3 eine Durchflussmenge bzw. einen Volumenstrom durch die Mediumzufuhrleitung 3 zu ändern. Es versteht sich, dass auch die in Fig. 1 bis Fig. 4 gezeigten Implantate 1 bzw. deren Mediumzufuhrleitungen 3 mit einem solchen Durchflussventil 15 ausgestattet sein können.

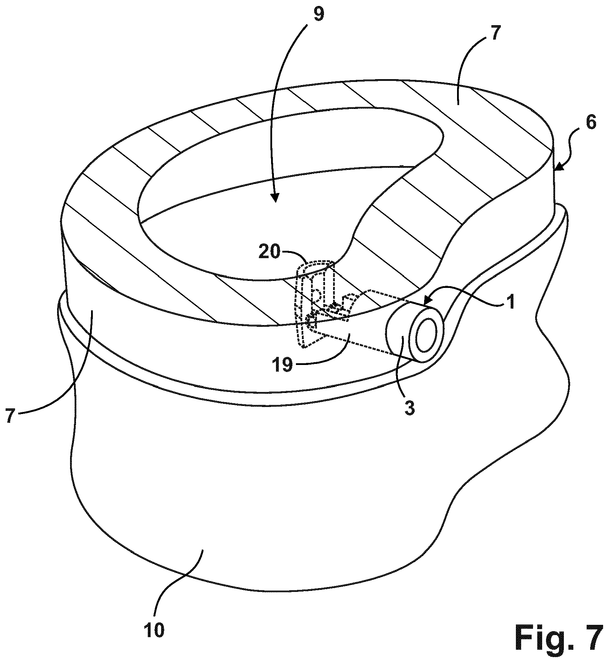

Fig. 7 zeigt eine schematische Darstellung einer weiteren Ausführungsvariante eines Implantates mit einem Erstreckungskörper in dessen Erstreckungszustand. Das Implantat 1 kann in gleicher Weise mit den gleichen Merkmalen und Wirkungen wie die Implantate 1 der Fig. 1 bis Fig. 6 aufgebaut sein. Im Unterschied zu den in Fig. 1 bis Fig. 6 gezeigten Implantaten 1 ist der Erstreckungskörper des Implantates 1 der Fig. 7 mit einem Basiskörper 19 und einem relativ zum Basiskörper 19 schwenkbaren bzw. kippbaren Blockierkörper 20 gebildet, um den Erstreckungskörper durch Schwenken bzw. Kippen des Blockierkörpers 20 relativ zum Basiskörper 19 von dessen Positionierungszustand in dessen Erstreckungszustand zu überführen. Der Blockierkörper 20 ist von einer Positionierungseinstellung in eine Erstreckungseinstellung relativ zum Basiskörper 19 schwenkbar, sodass in der Erstreckungseinstellung eine Länge des Erstreckungskörpers, insbesondere eine Länge quer zu einer Einführrichtung des Implantates 1 in die Bandscheibe 6, größer ist als in der Positionierungseinstellung. In Fig. 7 ist das Implantat 1 in dessen Erstreckungszustand gezeigt, in welchem der Blockierkörper 20 in die Erstreckungseinstellung geschwenkt ist. Das Implantat 1 ist dabei eingefügt in einen Hohlraum einer im Querschnitt dargestellten Bandscheibe 6 gezeigt. Eine hohe Robustheit ist erreichbar, wenn der Basiskörper 19 stoffschlüssig mit der Mediumzufuhrleitung 3 verbunden ist, wie dies in der Ausführungsvariante der Fig. 7 der Fall ist. Alternativ kann es günstig sein, wenn der Basiskörper 19 formschlüssig und/oder kraftschlüssig mit der Mediumzufuhrleitung 3 verbunden ist. Über die Mediumzufuhrleitung 3 bzw. eine Füllöffnung des Basiskörpers 19 kann Füllmedium in einen Hohlraum 9 der Bandscheibe 6 eingebracht werden, wobei der Blockierkörper 20 vorzugsweise eine Form bzw. Gestalt aufweist, um einen Druckverteilung in der Bandscheibe zu optimieren und/oder eine Druckkraft auf Deckplatten von an die Bandscheibe angrenzenden Wirbelkörpern 10 aufzubringen bzw. einen Wirbelkörperabstand wiederherzustellen und/oder eine Fixierung bzw. Positionierung des Implantates 1, insbesondere unter Bildung eines Formschlusses zwischen Implantat 1 und Anulus-Fibrosus 7, zu erreichen.

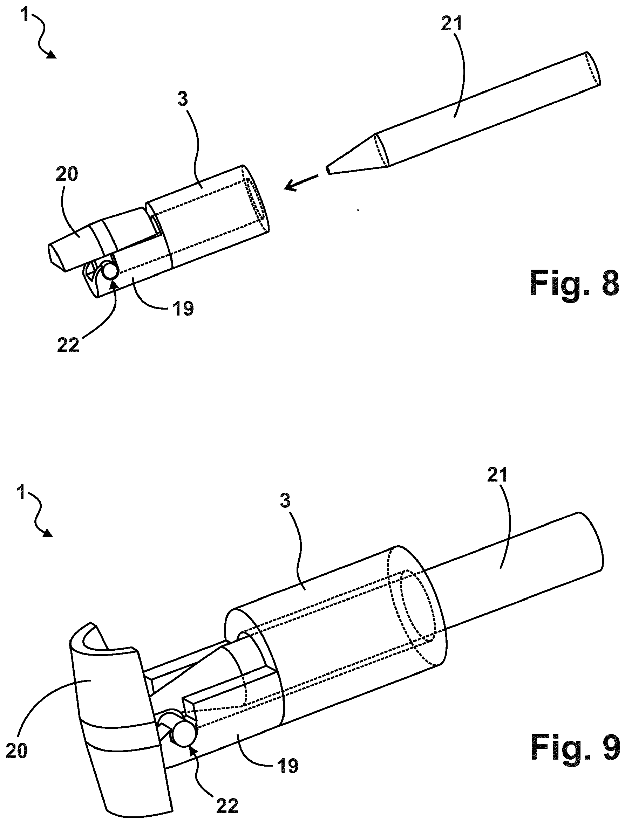

Fig. 8 zeigt eine schematische Darstellung des Implantates 1 der Fig. 7 mit dem Erstreckungskörper in dessen Positionierungszustand. Der Blockierkörper 20 ist hierfür in dessen Positionierungseinstellung am Basiskörper 19 angeordnet. Ersichtlich ist, dass das Implantat 1 dadurch eine für ein Einführen des Implantates 1 in die Bandscheibe 6 kompakte Größe bzw. Länge in Richtung quer zur Einführrichtung des Implantates 1 in die Bandscheibe 6 aufweist. Der Blockierkörper 20 ist mit einem Gelenk 22 mit dem Basiskörper 19 verbunden, wobei das Gelenk 22 vorzugsweise als Scharnier ausgebildet ist. Hierfür ist es praktikabel, wenn der Blockierkörper 20 eine Drehachse aufweist, welche in einer oder mehreren Drehachsenaufnahmen am Basiskörper 19 verschwenkbar bzw. kippbar gelagert ist, ersichtlich in Fig. 8 und Fig. 9. Weiter in Fig. 8 dargestellt ist ein Applikator 21, um den Blockierkörper 20 von der Positionierungseinstellung in die Erstreckungseinstellung zu überführen bzw. zu schwenken, indem der Applikator 21 gegen den Basiskörper 19 gedrückt bzw. gestoßen wird. Hierbei ist vorgesehen, dass der Applikator 21 über die Mediumzufuhrleitung 3 zum Blockierkörper 20 geführt wird, um den Blockierkörper 20 durch Anstoßen mit dem Applikator 21 in die Erstreckungseinstellung zu überführen bzw. relativ zum Basiskörper 19 zu schwenken. Für eine einfache Handhabung ist es praktikabel, wenn der Blockierkörper 20 ein Stoßsegment aufweist, welches üblicherweise als Erhebung einer Außenoberfläche des Blockierkörpers 20 ausgebildet ist, wobei die Außenoberfläche des Stoßsegmentes vorzugsweise als Abschnitt einer Rotationsfläche, beispielsweise als Abschnitt eines Rotationsellipsoides oder einer Kugel ausgebildet ist. Auf diese Weise kann durch ein Drücken bzw. Stoßen des Applikators 21 gegen das Stoßsegment, insbesondere wenn der Applikator 21 als Stab mit einer im Wesentlichen konusförmigen Spitze ausgebildet ist, eine Translationsbewegung des Stabes bzw. eine Druckkraft des Stabes effizient in eine Schwenkbewegung des Blockierkörpers 20 überführt werden.

Fig. 9 zeigt eine schematische Darstellung des Implantates 1 der Fig. 7 und Fig. 8 mit dem Erstreckungskörper in dessen Erstreckungszustand. Der Blockierkörper 20 ist hierfür in dessen Erstreckungseinstellung geschwenkt bzw. gekippt, sodass eine Länge des Erstreckungskörpers in Richtung quer zur Einführrichtung des Implantates 19 größer ist als im Positionierungszustand des Erstreckungskörpers. Wie in Fig. 9 ersichtlich, erfolgt das Schwenken bzw. Kippen des Blockierkörpers 20 in dessen Erstreckungseinstellung vorzugsweise durch Drücken bzw. Stoßen des Applikators 21 gegen den Blockierkörper 20.

In den vorstehend dargestellten Ausführungsvarianten kann der Erstreckungskörper, insbesondere der expandierbare Körper 2 bzw. Basiskörper 19 und/oder der Blockierkörper 20 vorteilhaft mit bzw. aus Drähten, insbesondere einem Drahtgeflecht oder Drahtgewebe, gebildet sein, wobei die Drähte aus einer bioresorbierbaren Mg-Zn-Ca-Legierung gebildet sind. Dadurch ist der Erstreckungskörper mit einer besonders ausgeprägten Belastbarkeit und Expansionsfähigkeit ausbildbar, wobei gleichzeitig eine hohe Biokompatibilität bzw. Bioverträglichkeit gewährleistet ist.

Auf diese Weise ist ein Implantat 1 mit Erstreckungskörper erreichbar, welches unter Ausnutzung der besonderen mechanischen Eigenschaften von Mg-Zn-Ca-Legierungen bei gleichzeitig hoher Bioverträglichkeit eine robuste, belastbare und heilungsoptimierte Behandlung einer Bandscheibe 6 ermöglicht.

## Patentansprüche

1. Implantat (1) zur Behandlung einer Bandscheibe (6), indem zumindest ein Teil der Bandscheibe (6) durch zumindest einen Teil des Implantates (1) ersetzt wird und/oder zumindest ein Teil des Implantates (1) in die Bandscheibe (6) eingeführt wird, wobei das Implantat (1) einen Erstreckungskörper aufweist, welcher von einem Positionierungszustand in einen Erstreckungszustand, in welchem der Erstreckungskörper in zumindest einer Richtung, insbesondere quer zu einer Einführrichtung des Implantates (1), eine größere Länge aufweist als im Positionierungszustand, überführbar ist, um den Erstreckungskörper im Positionierungszustand zumindest teilweise in einem Bandscheibenzwischenwirbelraum der Bandscheibe (6) zu positionieren und anschließend in den Erstreckungszustand zu überführen, **dadurch gekennzeichnet, dass** der Erstreckungskörper mit einer bioresorbierbaren Mg-Zn-Ca-Legierung gebildet ist.

2. Implantat (1) nach Anspruch 1, **dadurch gekennzeichnet, dass** der Erstreckungskörper in dessen Positionierungszustand zumindest teilweise durch eine Öffnung in einem Anulus-Fibrosus (7) der Bandscheibe (6) in einen Hohlraum (9) der Bandscheibe (6) einfügbar ist, um nach anschließendem Überführen des Erstreckungskörpers in dessen Erstreckungszustand mit dem Erstreckungskörper einen Nucleus-Pulposus (8) der Bandscheibe (6) zumindest teilweise zu ersetzen und/oder die Öffnung im Anulus-Fibrosus (7) zu verschließen.

3. Implantat (1) nach Anspruch 1 oder 2, **dadurch gekennzeichnet, dass** der Erstreckungskörper mit einem expandierbaren Körper (2) ausgebildet ist, welcher im Erstreckungszustand ein größeres Volumen aufweist als im Positionierungszustand.

4. Implantat (1) nach Anspruch 3, **dadurch gekennzeichnet, dass** der expandierbare Körper (2) zumindest einen mit einem fließfähigem, insbesondere flüssigen, gelartigen und/oder gasförmigen, Füllmedium befüllbaren Füllraum aufweist, um den expandierbaren Körper (2) durch Befüllen des Füllraumes mit Füllmedium vom Positionierungszustand in den Expansionszustand zu überführen.

5. Implantat nach einem der Ansprüche 1 bis 4, **dadurch gekennzeichnet, dass** der Erstreckungskörper einen Basiskörper (19) und zumindest einen relativ zum Basiskörper (19) beweglichen, insbesondere schwenkbaren, Blockierkörper (20) aufweist, um den Erstreckungskörper durch Bewegen des Blockierkörpers (20) relativ zum Basiskörper (19) vom Positionierungszustand in den Erstreckungszustand zu überführen.

6. Implantat (1) nach einem der Ansprüche 2 bis 5, **dadurch gekennzeichnet, dass** das Implantat (1) zumindest eine mit dem Erstreckungskörper verbundene Mediumzufuhrleitung (3) aufweist, über welche Füllmedium dem Hohlraum (9) der Bandscheibe (6) bzw. gegebenenfalls dem zumindest einen Füllraum zuführbar ist.

7. Implantat (1) nach Anspruch 6, **dadurch gekennzeichnet, dass** die Mediumzufuhrleitung (3) mittels kraftschlüssiger und/oder formschlüssiger Verbindung mit dem Erstreckungskörper, insbesondere lösbar, verbunden ist.

8. Implantat (1) nach Anspruch 6 oder 7, **dadurch gekennzeichnet, dass** das Implantat (1) zumindest ein steuerbares Durchflussventil (15) aufweist, um eine Durchflussmenge, insbesondere einen Volumenstrom, eines über eine Füllöffnung des Erstreckungskörpers dem Hohlraum (9) der Bandscheibe (6), gegebenenfalls Füllraum, zugeführten Füllmediums zu steuern.

9. Implantat (1) nach einem der Ansprüche 6 bis 8, **dadurch gekennzeichnet, dass** das Implantat (1) zumindest eine Rückschlageinrichtung aufweist, welche ein Zuführen von Füllmedium über eine Füllöffnung des Erstreckungskörpers in den Hohlraum (9) der Bandscheibe (6), gegebenenfalls Füllraum des expandierbaren Körpers (2), erlaubt, aber einen Austritt von Füllmaterial aus dem Hohlraum (9), gegebenenfalls Füllraum, über die Füllöffnung begrenzt oder verhindert.

10. Implantat (1) nach einem der Ansprüche 6 bis 9, **dadurch gekennzeichnet, dass** die Mediumzufuhrleitung (3) und/oder gegebenenfalls das Durchflussventil (15) und/oder gegebenenfalls die Rückschlageinrichtung mit einem bioresorbierbaren Material, insbesondere einer bioresorbierbaren Mg-Zn-Ca-Legierung, gebildet ist.

11. Implantat (1) nach einem der Ansprüche 1 bis 10, **dadurch gekennzeichnet, dass** die bioresorbierbare Mg-Zn-Ca-Legierung (in Gew.-%)
mehr als 0,0 % bis 1,5 % Zink,
mehr als 0,0 % bis 1,0 % Calcium,
optional mehr als 0,0 % bis 1,2 % Ytterbium,
Rest Magnesium und herstellungsbedingte Verunreinigungen
aufweist.

12. Implantat (1) nach Anspruch 10 oder 11, **dadurch gekennzeichnet, dass** am Einsatzort des Implantates (1) das bioresorbierbare Material der Mediumzufuhrleitung (3) und/oder gegebenenfalls das bioresorbierbare Material des Durchflussventiles (15) und/oder gegebenenfalls das bioresorbierbare Material der Rückschlagvorrichtung eine höhere Degradationsgeschwindigkeit als die bioresorbierbare Mg-Zn-Ca-Legierung des Erstreckungskörpers aufweist.

13. Implantat (1) nach einem der Ansprüche 1 bis 12, **dadurch gekennzeichnet, dass** der Erstreckungskörper mit zumindest einem mit der bioresorbierbaren Mg-Zn-Ca-Legierung gebildeten Draht, insbesondere Drahtgeflecht oder Drahtgewebe, ausgebildet ist.

14. Implantat (1) nach einem der Ansprüche 1 bis 13, **dadurch gekennzeichnet, dass** der Erstreckungskörper in dessen Erstreckungszustand an gegenüberliegenden Seiten des Erstreckungskörpers im Wesentlichen parallel zueinander angeordnete, insbesondere ebene, Außenflächen aufweist, um in einer Einsatzposition des Implantates (1) mit den Außenflächen eine Druckkraft auf Deckplatten von an die Bandscheibe (6) angrenzenden Wirbeln bzw. Wirbelkörpern (10) aufzubringen.

15. Verfahren zur Herstellung eines Implantates (1) nach einem der Ansprüche 1 bis 14, **dadurch gekennzeichnet, dass** das Implantat (1) bzw. ein Teil des Implantates (1) mit einem additiven Fertigungsverfahren, bevorzugt einem 3-D-Druck-Verfahren, hergestellt ist.
